# EUROPEAN PATENT APPLICATION

(11) **EP 1 435 243 A2**
(43) Date of publication of application: **07.07.2004**
(21) Application number: 04008347.9
(22) Date of filing: 12.11.1999
(51) Int. Cl.: A61K 38/18, A61P 35/00

(54) **Methods of alleviating cancer symtoms**

(30) Priority: 13.11.1998 US 191239
(62) Divisional of application: 99958892.4
(71) Applicant: Curis, Inc., Cambridge, MA 02138 (US)
(72) Inventor: Sampath, Kuber T., Holliston, MA 01746 (US); Cohen, Charles M., Weston, MA 02193 (US); Rueger, David C., Southborough, MA 01772 (US)
(74) Representative: MacLean, Martin Robert

(57) **Abstract**

The present invention provides use of a morphogen for the manufacture of a medicament for alleviating cancer symptoms, wherein the morphogen inhibits cancer cell growth and is selected from the group consisting of: BMP-9, BMP-11, BMP-14, BMP-15, GDF-8, GDF-9, GDF-11, ADMP and NEURAL.

## Description

### BACKGROUND OF THE INVENTION

Cancer is a major cause of death throughout the world. A characteristic feature of cancer is cell growth not regulated by external signals. Cancer cells also may develop the capacity to metastasize and to colonize distant sites. See generally HARRISON'S PRINCIPLES OF INTERNAL MEDICINE, 505 (Fauci *et al*. eds., 14th ed., 1998). Cancer is most common in tissue that has a relatively rapid cell turnover rate. In most cases, two types of cells exist in those tissue types: dividing progenitor cells and non-dividing differentiated cells. The term cancer refers to both benign growths in which cells divide without regulation, but are unable to metastasize, and malignant growths in which cells divide without regulation, and are able to metastasize.

In the absence of a disease, such as cancer, or trauma, once a cell has acquired a particular differentiated state, its fate is typically irreversible. Morphogens are known to induce the proliferation and differentiation of progenitor cells in numerous tissues. Morphogens include members of the family of bone morphogenetic proteins (BMPs) identified by their ability to induce ectopic, endochondral bone morphogenesis. The morphogens, also referred to as osteogenic proteins, generally are classified as a subgroup of the TGF-β superfamily of growth factors. Hogan, *Genes & Development 10*: 1580-1594 (1996). Members of that superfamily include the mammalian osteogenic protein-1 (OP-1, also known as BMP-7, and the *Drosophila* homolog 60A), osteogenic protein-2 (OP-2, also known as BMP-8), osteogenic protein-3 (OP-3), BMP-2 (also known as BMP-2A or CBMP-2A, and the *Drosophila* homolog DPP), BMP-3, BMP-4 (also known as BMP-2B or CBMP-2B), BMP-5, BMP-6 and its murine homolog Vgr-1, BMP-9, BMP-10, BMP-11, BMP-12, GDF3 (also known as Vgr2), GDF8, GDF9, GDF10, GDF11, GDF12, BMP-13, BMP-14, BMP-15, GDF-5 (also known as CDMP-1 or MP52), GDF-6 (also known as CDMP-2), GDF-7 (also known as CDMP-3), the Xenopus homolog Vgl and NODAL, UNIVIN, SCREW, ADMP, and NEURAL.

Morphogens share common structural features. The mature form results from processing through a "pro-form" to yield a mature polypeptide containing a carboxyl terminal active domain of approximately 97-106 amino acids. All morphogens share a conserved pattern of cysteines in that domain. The active form is either a disulfide-bonded homodimer of a single superfamily member or a heterodimer of two different members. See, *e.g*., Massague, *Annu. Rev. Cell Biol. 6*: 597 (1990); Sampath *et al., J. Biol. Chem. 265*: 13198 (1990).

Efforts to alleviate the symptoms of cancer, totally or partially, have often focused on physical excision of cancerous tissue, radiation-induced destruction of cancerous tissue, and chemical-induced destruction of cancerous tissue. While these modes of treatment have given hope to those afflicted with cancer, they have drawbacks in many instances including invasiveness (in the case of physical excision), cytotoxicity, production of serious side effects in the treated subject, limited therapeutic windows, and limited ability to completely destroy a cancerous tissue. Additionally, some less traditional efforts have been made to develop biological agents that regulate the host's own biological machinery to respond to cancer cells. Moreover, not all anti-cancer agents are equally efficacious on all cell types. Thus, a need exists for anti-cancer agents that are capable of alleviating the symptoms of cancer that are effective at a broader range of cell types (or, alternatively, at new or different subsets of cells types) and have fewer problems associated with their use than current anti-cancer agents.

### SUMMARY OF THE INVENTION

The present invention provides compositions and methods that inhibit or prevent the unchecked growth of cancer cells or stimulate differentiation of cancer cells. Many cancers are characterized by cells that display unregulated growth with or without the ability to metastasize to additional sites throughout the body. One method to treat a cancer to alleviate the symptoms of the cancer is to inhibit the unregulated proliferation of cancerous cells. Morphogens (also known as bone morphogenic proteins, "BMPs," or "morphogenic proteins") are a family of proteins capable of altering cellular phenotype in many different cell types. In particular; morphogens are capable of stimulating, maintaining, or regenerating a differentiated cell phenotype. It has now been discovered that exposing cancer cells to morphogens inhibits cancer cell growth and causes such cells to differentiate away from the cancerous phenotype. Thus, administering a morphogen can influence cancer cell fate and, in turn, alleviate the symptoms of cancer.

The present invention is an alternative approach to methods currently being used to treat cancer and alleviate symptoms of this disease. Methods of the invention also reduce the side effects typically observed with conventional chemotherapy methods for treating cancer.

Accordingly, a preferred embodiment of the present invention is a method of treating cancer to alleviate the symptoms of cancer by administering a morphogen to a patient having cancer. Morphogens are dimeric proteins having an amino acid sequence of either at least 70% homology with the C-terminal seven-cysteine skeleton of human OP-1 which includes residues 330-431 of SEQ ID NO: 2, or an amino acid sequence having at least 60% identity with this C-terminal seven-cysteine skeleton of human OP-1, SEQ ID NO: 3, SEQ ID NO: 4, SEQ ID NO: 5, SEQ ID NO: 6, or SEQ ID NO: 7, which inhibit the growth of cancer cells. Morphogens useful in the invention include, but are not limited to, OP-1, OP-2, OP-3, BMP-2, BMP-3, BMP-3b, BMP-4, BMP-5, BMP-6, BMP-9, BMP-10, BMP-11, BMP-14, BMP-15, DPP, Vgl, Vgr-1, 60A protein, CDMP-1, CDMP-2 (BMP-13), CDMP-3 (BMP-12), GDF-1, GDF-3, GDF-5, GDF6, GDF-7, GDF-8, GDF-9, GDF-10, GDF-11, GDF-12, NODAL, UNIVIN, SCREW, ADMP, NEURAL, and morphogenically active amino acid sequence variants thereof. In another aspect of the invention, the morphogen is non-covalently associated with at least one pro-domain polypeptide selected from the pro-domains of OP-1, OP-2,60A, GDF-1, BMP-2A, BMP-2B, DPP, Vgl, Vgr-1, BMP-3, BMP-5, and BMP-6. In another aspect of this invention, the morphogen is non-covalently associated with more than one pro-domain polypeptide of a morphogen useful in the compositions and methods described herein.

In another aspect of the invention, the morphogen is administered via a pharmaceutically acceptable carrier or vehicle. The carrier or vehicle may be a physiologically acceptable aqueous solution such as a saline or buffer solution (*e*.*g*., a phosphate buffered saline solution). The carrier or vehicle comprises a liposome or microsphere that is biocompatible, biodegradable and/or bioerodable. Alternatively, the carrier or vehicle comprises a biocompatible matrix, which is biodegradable or bioerodable. Compounds and substances useful as matrices in the invention include, without limitation, proteinaceous materials, (*e.g*., collagen), mono- and polysaccharides and derivatives thereof, (*e*.*g*., carboxymethyl cellulose and hyaluronic acid), synthetic polymers (*e*.*g*., polyglycolic and polylactic acid polymers or co-polymers), calcium-containing compounds, (*e*.*g*., hydroxyapatite or tricalcium phosphate), and combinations thereof.

In another aspect of the invention, the step of administering a morphogen comprises delivering to a cancer cell a cell that is capable of expressing morphogen. In another aspect of the invention, the step of administering a morphogen comprises providing a non-infectious, non-integrating DNA encoding a morphogen that is operably linked to a promoter which directs expression of the morphogen in cells of a particular tissue. In another aspect of the invention, an enhancer element is in operative association with the promoter. In yet another embodiment, DNA encoding a morphogen is operably linked to a promoter. The DNA is associated with a liposome, microsphere, or viral particle that facilitates uptake of the DNA and expression of the morphogen by cancer cells or by neighboring non-cancer cells. Viral particles useful in transducing DNA encoding morphogen into cancer cells or neighboring non-cancer cells include adeno-associated virus particles.

In a preferred embodiment, symptoms of cancer are alleviated in adrenal cancer, anus cancer, bladder cancer, bone cancer, brain cancer, breast cancer, cervix cancer, colon cancer, corpus cancer, endocrine cancer, esophageal cancer, fallopian tube cancer, fat cell cancer, gallbladder cancer, gastrointestinal tract cancer, germ cell tumors, kidney cancer, leukemia, liver cancer, lymphoma, lung cancer, muscle cancer, nervous system cancer, ocular tissue cancer, oral cancer (*e*.*g*., tongue, oral epithelial tissue), ovarian cancer, pancreatic cancer, prostate cancer (androgen-dependent and -independent), rectal cancer, skin cancer, small intestine cancer, soft tissue cancer, stomach cancer, teratocarcinoma (teratomas), testicular cancer, thyroid cancer, ureteral cancer, urinary cancer, uterine cancer, and metastatic forms thereof cancer of unknown primary site.

In a preferred embodiment of the invention, a cancer is treated by administering to a cancer patient a morphogen to inhibit the unregulated proliferation of cancer cells or to stimulate differentiation of cancer cells away from the cancer phenotype. In another aspect of the invention, morphogens are used to treat cancer to alleviate other symptoms of cancer comprising abnormal bleeding, abnormal bodily function, abnormal cell morphology, abnormal enzyme levels, abnormal hormone levels, abnormal oncofetal antigen levels, abnormal tissue growth, abnormal tissue mass, abnormal tumor-associated protein levels, altered neurologic function, altered neurologic structure, angiogenesis, diarrhea, effusions, fatigue, fever, lesions, malnutrition, metastasis, nausea, obstruction of a bodily passageway, opportunistic infection, pain, poor Karnofsky performance status, presence of cell-surface markers, presence of histological cancer markers, presence of intracellular markers, presence of molecular markers, tumor invasion, urinary frequency, weight loss, and combinations thereof. Inhibition of proliferation may be measured, for example, by increased survival rates, decreased tumor size, or tumor regression.

In another aspect of the invention, the expression of bone morphogenic protein receptors (BMPRs) on targeted cancer cells provides a means for determining tissue likely to respond to morphogen treatment. In a preferred embodiment, targeted malignant cells in a patient are pre-screened for expression of BMPRs to enable the selection of patients with a high probability of responding to a given BMP treatment.

In another aspect of this invention, morphogens inhibit cellular growth as measured, for example, by cellular incorporation of [³H]-thymidine in an *in vitro* assay, shrinkage in tumor size, or decrease in expression of cancer-associated antigens. In another aspect of the invention, morphogen stimulates cancer cells to differentiate away from the cancer phenotype, for example, as indicated by decreased expression of cancer-associated antigens, a change in cell morphology, or an increased expression of antigenic or biochemical markers associated with a preferred differentiated cell phenotype. In another aspect of this invention, prior to administering morphogens, the affected tissue is removed from the host. After administering morphogen in an *ex vivo* protocol, the affected tissue is replaced into the host. In methods of the invention, morphogen may be administered in combination with one or more non-morphogenic agents, such as known anti-cancer agents, analgesics, or anti-inflammatory compounds.

Another preferred embodiment of the invention comprises a method of treating cancer to alleviate one or more symptoms of the cancer by administering a morphogenically active amino acid variant of a naturally occurring morphogen. Such variants include naturally occurring allelic variants, conservative amino acid substitution variants, peptide fragment variants, and conservative amino acid substitution variants of peptide fragments that inhibit cancer cell growth.

Another preferred embodiment of the invention comprises a method to alleviate the symptoms of a cancer by administering morphogens which are dimeric proteins having an amino acid sequence of either at least 70% homology with the C-terminal seven-cysteine skeleton of human OP-1 which includes residues 330-431 of SEQ ID NO: 2, an amino acid sequence having at least 60% identity with this C-terminal seven-cysteine skeleton of human OP-1, SEQ ID NO: 3, SEQ ID NO: 4, SEQ ID NO: 5, SEQ ID NO: 6, or SEQ ID NO: 7, which inhibit cellular incorporation of [³H]-thymidine in an *in vitro* assay. Another aspect of the invention comprises a step of administering a substance that is a bone morphogenic protein or a morphogenically active amino acid variant of a bone morphogenic protein. Variants include a naturally occurring allelic variant of a bone morphogenic protein, a conservative amino acid substitution variant of a bone morphogenic protein, a peptide fragment variant of a bone morphogenic protein, and a conservative amino acid substitution variant of a peptide fragment variant.

The invention will be understood further from the following drawings, description, and claims.

### BRIEF DESCRIPTION OF THE DRAWINGS

FIGS. 1A-1M are tabular alignments of the amino acid sequences of various naturally occurring morphogens with a preferred reference sequence of human OP-1, residues 330-431 of SEQ ID NO: 2.
FIGS. 2A-2B are tabular presentations of alternative amino acids for "Xaa" positions in generic sequences SEQ ID NOS: 4, 5, and 8 that represent amino acid variations in known morphogens.
FIGS. 3A-3C are tabular presentations of alternative amino acids for "Xaa" positions in generic sequence SEQ ID NOS: 6, 7, and 9 that represent amino acid. variations in known morphogens.
FIG. 4 is a tabular presentation of alternative amino acids for "Xaa" positions in generic sequence SEQ ID NO: 3 that represents amino acid variations in several identified allelic and phylogenetic variants of OP-1 and OP-2.
FIGS. 5A-5B illustrates the ability of morphogen (OP-1) to induce undifferentiated NG108 cells (FIG. 5A) to undergo differentiation of neural morphology (FIG. 5B).
FIGS. 6A-6D show the effect of morphogen (OP-1) on human embryo carcinoma cell redifferentiation.
FIG. 7 shows the effect ofmorphogen (BMP 6 or OP-1 (BMP 7)), Activin or TGF-β1 on cell growth of unstimulated androgen responsive prostate cancer LNCaP cells.
FIG. 8 shows the effect of morphogen (BMP-6 or OP-1 (BMP-7)), Activin or TGF-β1 on cell growth of stimulated androgen-responsive prostate cancer LNCaP cells.
FIG. 9 shows the effect of follistatin on the bioactivity of morphogens (BMP-6 or OP-1 (BMP-7)) or Activin in androgen-responsive prostate cancer LNCaP cells.
FIGS. 10A-10B show the effect of morphogen (OP-1) or TGF-β1 on cell growth of estrogen responsive human breast cancer MCF-7 cells in the absence (FIG. 1 0A) or in the presence (FIG. 10B) of 5% serum containing medium.
FIGS. 11A-11B show the effect of morphogen (OP-1) on cell growth of 17-β estradiol stimulated or unstimulated estrogen responsive human breast cancer MCF-7 cells (FIG. 11A) and the effect of TGF-β1 on cell growth of 17-β estradiol stimulated or unstimulated estrogen responsive human breast cancer MCF-7 cells (FIG. 11B).
FIG. 12 shows the effect of morphogen (OP-1), tamoxifen, or both morphogen (OP-1) and tamoxifen combined on cell growth of estrogen-responsive human breast cancer MCF-7 cells.
FIG. 13 shows the effect of morphogen (OP-1) on cell growth of human glioblastoma A-172 cells.
FIG. 14 shows the effect of morphogen (OP-1) or TGF-β2 on cell growth of human lung carcinoma A-549 cells.
FIG. 15 shows the expression of three BMPRs in murine prostate cancer cell lines E4 and F11.
FIG. 16 shows the effect of morphogen (BMP-6) on cell growth of murine prostate cancer cell lines E4 and F11.
FIG. 17 shows the effect of morphogen (BMP-6) on tumor volume in mice with prostate cancer xenografts.
FIG. 18 shows the effect of morphogen (BMP-6) on tumor volume in mice with prostate cancer xenografts, corrected for surviving animals.
FIGS. 19A-19B shows necrosis and diffuse inflammation in tumors treated with morphogen (BMP-6) as compared to control tumors.

### DETAILED DESCRIPTION OF THE INVENTION

The present invention provides compositions and methods that inhibit the unchecked proliferation of cancer cells, stimulate differentiation of cancer cells away from their particular cancer phenotype, or alleviate various other symptoms of a cancer. Many cancers are characterized by cells that display unregulated growth with or without the ability to metastasize to additional sites throughout the body. One method to alleviate the symptoms of cancer is to stop cancerous cells from growing and dividing. Morphogens are capable of differentiating progenitor cells (*i.e*., dividing cells). Exposure to morphogens inhibits growth of cancer cells. Thus, administering a morphogen influences cancer cell fate and, in turn, alleviates various characteristic symptoms of cancer.

BMP signal transduction follows the paradigm established by TGF-β signaling. As with TGF-β, BMPs signal through an interaction with a heteromeric complex of membrane receptors, type I and type II. Specifically, ligand binding results in cross-phosphorylation of type I receptor by type II receptor; type II receptor, in turn, propagates BMP signaling. *See*, Yamashita *et al*., *Bone 19*: 569-574 (1996). *In vitro* experiments have shown that all members of BMP that belong to the TGF-β superfamily bind to BMPR-II in combination with BMPR-IA or IB. *See*, Yamashita *et al., supra.* Thus, BMPR-IA, BMPR-IB, and BMPR-II appear to be BMP-specific receptors, and are likely to mediate morphogen inhibition of cancer cell growth.

### I. USEFUL MORPHOGENS

In its mature, native form, natural-sourced morphogen is a glycosylated dimer, typically having an apparent molecular weight of about 30-36 kDa as determined by SDS-PAGE. When reduced, the 30 kDa protein gives rise to two glycosylated peptide subunits having apparent molecular weights of about 16 kDa and 18 kDa. In the reduced state, the protein has no detectable osteogenic activity. The unglycosylated protein, which also has osteogenic activity, has an apparent molecular weight of about 27 kDa. When reduced, the 27 kDa protein gives rise to two unglycosylated polypeptide chains, having molecular weights of about 14 kDa to 16 kDa. Typically, the naturally occurring osteogenic proteins are translated as a precursor, having an N-terminal signal peptide sequence typically less than about 30 residues, followed by a "pro" domain that is cleaved to yield the mature C-terminal domain. The signal peptide is cleaved rapidly upon translation, at a cleavage site that can be predicted in a given sequence using the method of Von Heijne, *Nucleic Acids Res*. *14*: 4683-4691 (1986). The pro-domain typically is about three times larger than the frilly processed mature C-terminal domain.

Morphogens useful in the methods of the invention are defined by an amino acid sequence having at least 70% homology with the C-terminal seven-cysteine skeleton of human OP-1, residues 330-431 of SEQ ID NO: 2, or having at least 60% identity with this C-terminal seven-cysteine skeleton of human OP-1, and having the ability to stimulate endochondral bone formation in the bioassay described by Sampath and Reddi (*Proc. Natl*. *Acad. Sci. USA 80*: 659 1-6595 (1983) and U.S. Patent No. 4,968,590), the disclosures of which are incorporated by reference herein. Morphogens also include substances capable of binding morphogen receptors, capable of inducing other morphogens, and capable of agonist-type activity at morphogen receptors. These substances may be assayed for morphogenic capabilities by methods known in the art.

Preferred morphogens include those comprising the C-terminal 96 or 102 amino acid sequences of DPP (from *Drosophila*), Vgl (from Xenopus), Vgr-1 (from mouse), the OP-1 and OP-2 proteins (See U.S. Patent No. 5,011,691 and Oppermann *et al*.), as well as the proteins referred to as BMP-2, BMP-3, BMP-4 (*See* W088/00205, U.S. Patent No. 5,013,649 and W091/18098), BMP-5 and BMP-6 (*See* W090/1 1366, PCT/U590/0 1630), BMP-8 and BMP-9. Other proteins useful in the practice of the invention include active forms of OP-1, OP-2, OP-3, BMP-2, BMP-3, BMP-4, BMP-5, BMP-6, BMP-9, GDF-5, GDF-6, GDF-7, DPP, Vgl, Vgr, 60A protein, CDMP-1, CDMP-2 (BMP-13), CDMP-3 (BMP-12),GDF-1, GDF-3, GDF-5, GDF6, GDF-7, EMP-10, BMP-11, BMP-15, UNIVIN, NODAL, SCREW, ADMP or NEURAL and amino acid sequence variants thereof. In a currently preferred embodiment, osteogenic proteins include any one of: OP-1, OP-2, OP-3, BMP-2, BMP-4, BMP-5, BMP-6, BMP-9, and amino acid sequence variants and homologs thereof, including species homologs, thereof. Publications disclosing the OP-1 and OP-2 sequences, as well as their chemical and physical properties, include U.S. Patent Nos. 5,011,691 and 5,266,683, each incorporated by reference herein.

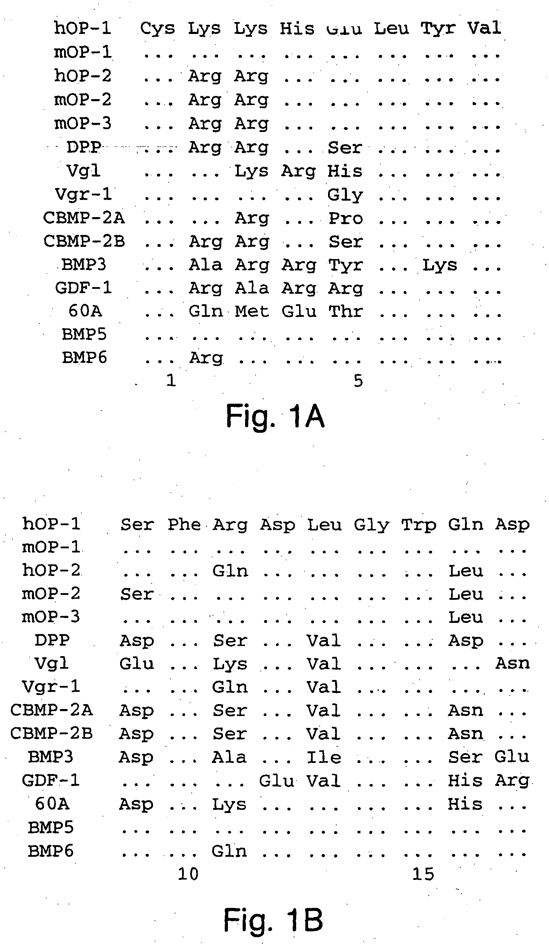

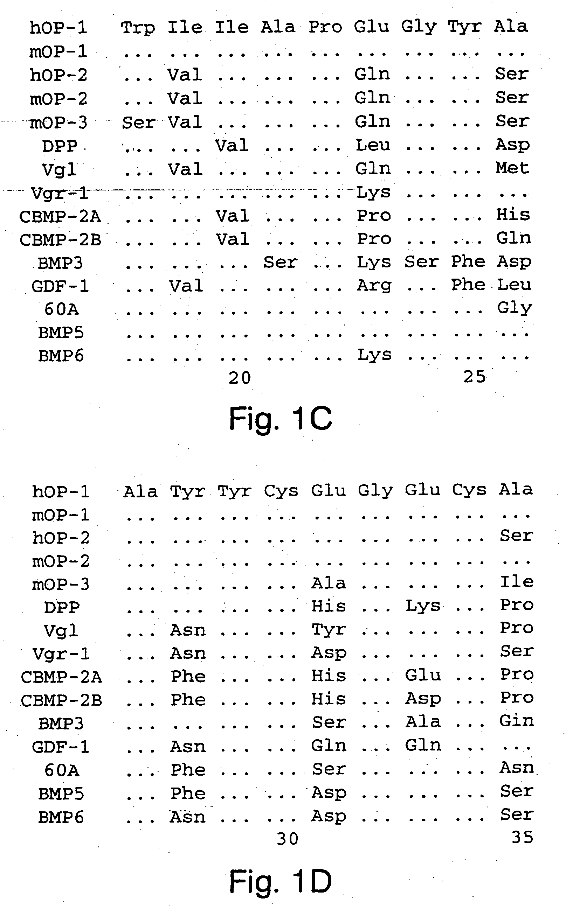

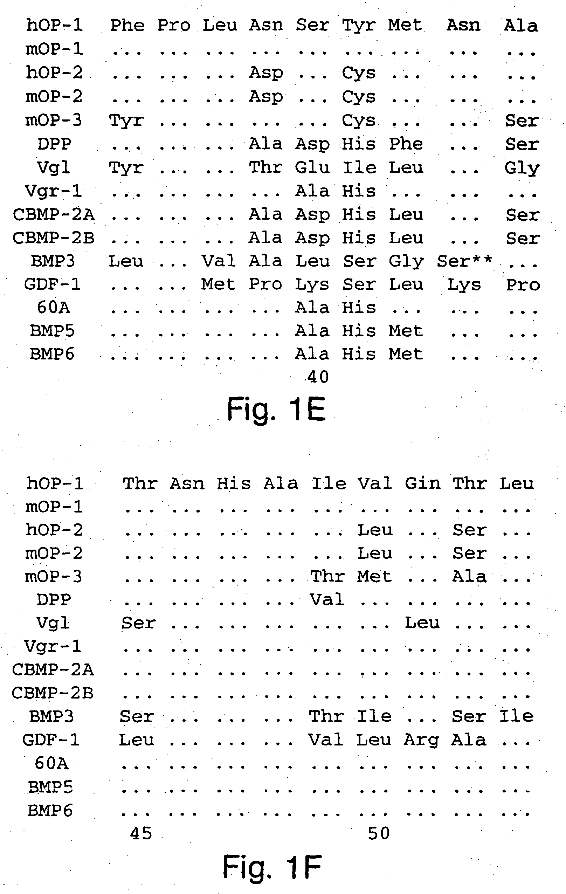

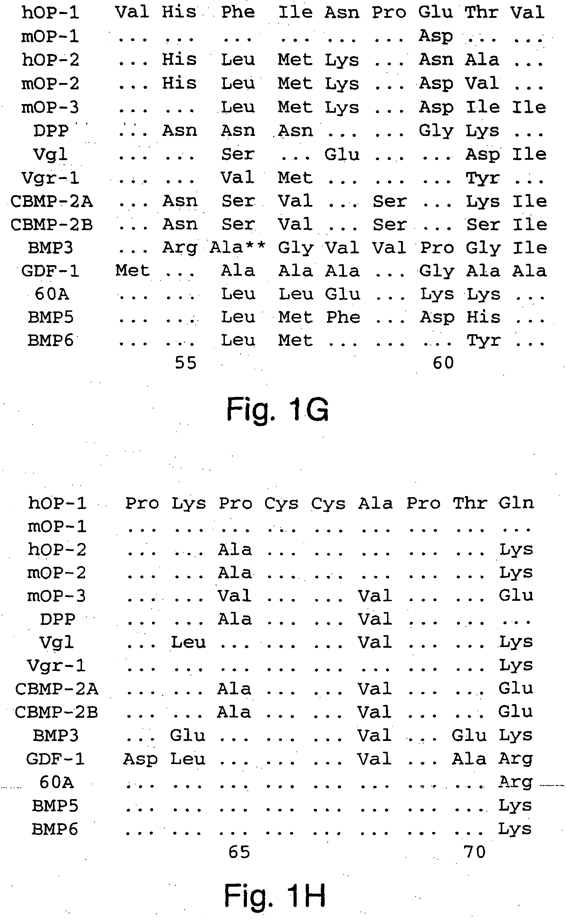

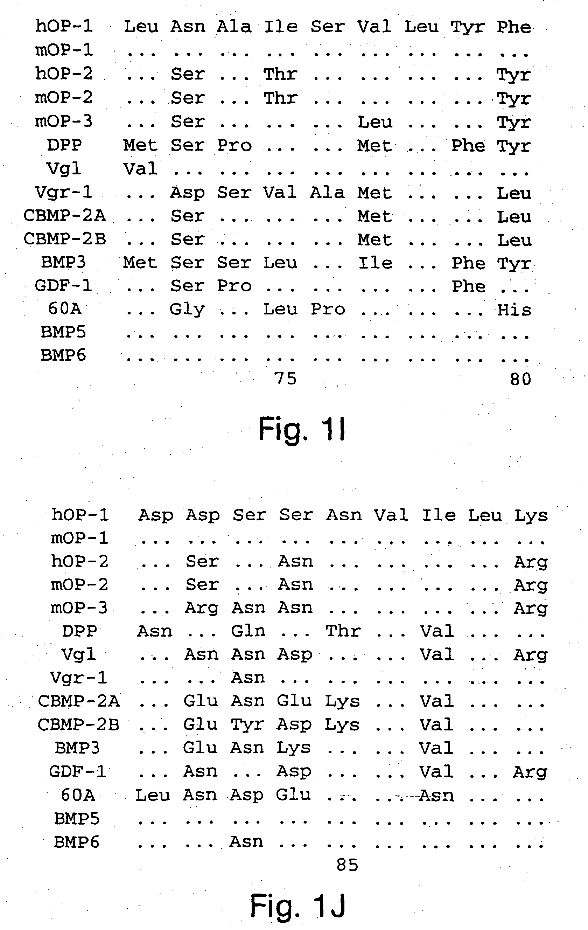

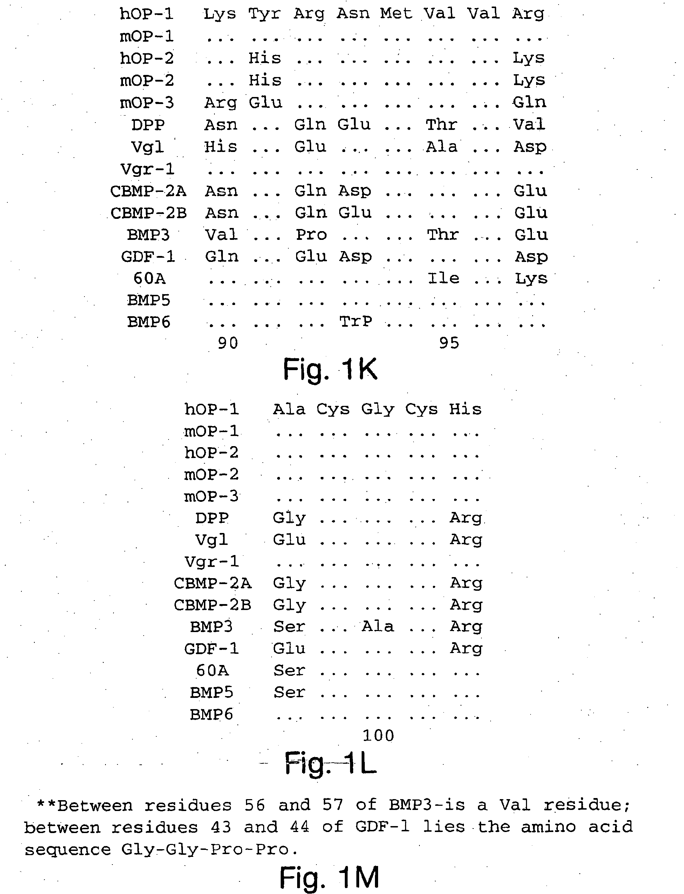

In preferred embodiments, morphogens for use in methods of the invention include proteins having at least 70% homology with the amino acid sequence of the C-terminal seven cysteine skeleton of human OP-1, SEQ ID NO: 2, and having the ability to induce endochondral bone formation in the Reddi and Sampath assay. Compounds that meet these requirements are considered functionally equivalent to a known response morphogen. To determine whether a candidate peptide is functionally equivalent to a reference morphogen, the candidate amino acid sequence and the reference morphogen amino acid sequence are aligned. The first step for performing an alignment is to use an alignment tool, such as the dynamic programming algorithm described in Needleman *et al, J. Mol. Biol. 48*: 443 (1970), and the Align Program, a commercial software package produced by DNAstar, Inc., the teachings of which are incorporated by reference herein. After the initial alignment is made, it is then refined by comparison to a multiple sequence alignment of a family of related proteins, such as those shown in FIGS. 1A through 1M, which is a multiple sequence alignment of a family of known morphogens, including hOP-1. Once the alignment between the candidate and reference sequences is made and refined, a percent homology score is calculated. The individual amino acids of each sequence are compared sequentially according to their similarity to each other. Similarity factors include similar size, shape and electrical charge. One particularly preferred method of determining amino acid similarities is the PAM25O matrix described in Dayhoff *et al*., 5 ATLAS OF PROTEIN SEQUENCE AND STRUCTURE 345-352 (1978 & Supp.), incorporated by reference herein. A similarity score is first calculated as the sum of the aligned pairwise amino acid similarity scores. Insertions and deletions are ignored for the purposes of percent homology and identity. Accordingly, gap penalties are not used in this calculation. The raw score is then normalized by dividing it by the geometric mean of the scores of the candidate compound and the seven cysteine skeleton of hOP-1. The geometric mean is the square root of the product of these scores. The normalized raw score is the percent homology.

In an alternative preferred embodiment, a candidate peptide that is functionally equivalent to a reference morphogen has an amino acid sequence that shares at least 60% amino acid identity with the reference sequence and has the ability to induce endochondral bone formation in the Reddi and Sampath assay. Sixty percent amino acid identity means that any 60% of the candidate peptide amino acids when aligned with the corresponding amino acids in the reference sequence are identical. Any one or more of the naturally occurring or biosynthetic morphogens disclosed herein may be used as a reference sequence to determine whether a candidate sequence falls within the morphogen family. In a preferred embodiment, the reference sequence is the C-terminal seven-cysteine skeleton sequence of human OP-1 as shown in SEQ ID NO: 2. Examples of conservative substitutions for use in the above calculations include the substitution of one amino acid for another with similar characteristics, *e.g*., substitutions within the following groups are well-known: (a) valine, glycine; (b) glycine, alanine; (c) valine, isoleucine, leucine; (d) aspartic acid, glutamic acid; (e) asparagine, glutamine; (f) seine, threonine; (g) lysine, arginine, methionine; and (h) phenylalanine, tyrosine. The term "conservative variant" or "conservative variation" also includes the use of a substituted amino acid in place of an unsubstituted parent amino acid in a given polypeptide chain, provided that antibodies having binding specificity for the resulting substituted polypeptide chain also have binding specificity (i.e., "crossreact" or "immunoreact") with the unsubstituted or parent polypeptide.

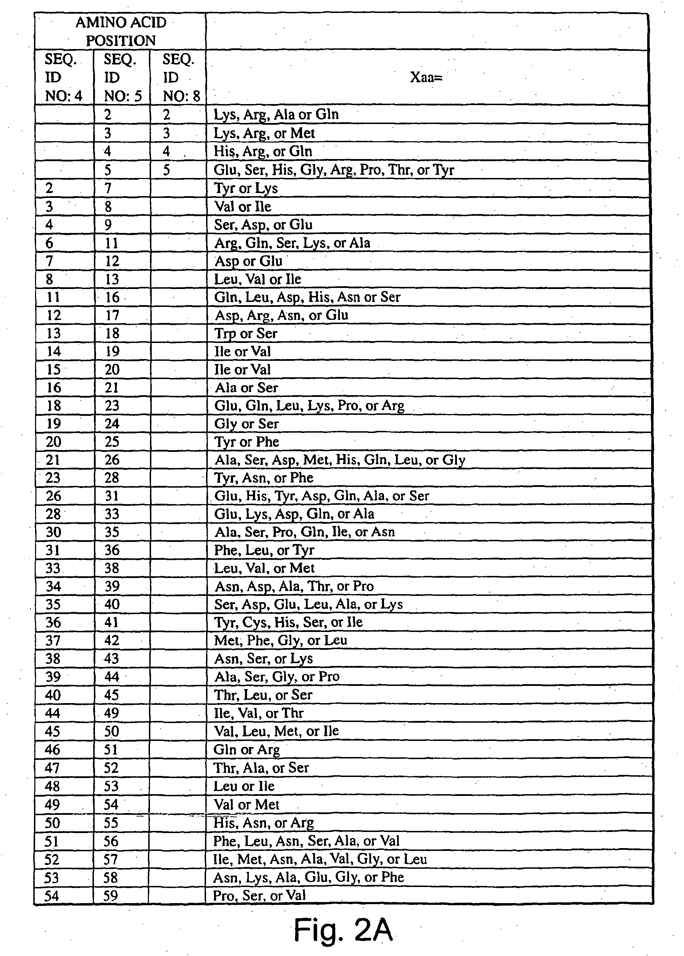

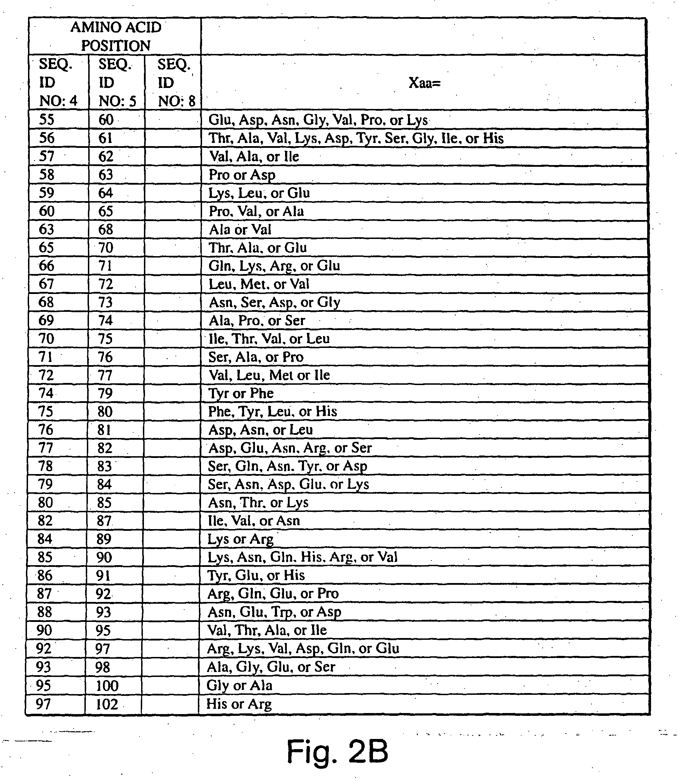

In a preferred embodiment, morphogens useful in the present invention are defined by a generic amino acid sequence that represents variations in known morphogens. For example, SEQ ID NOS: 4 and 5 encompass observed variations between preferred morphogens, including OP-1, OP-2, OP-3, CBMP-2A, CBMP-2B, BMP-3, 60A, DPP, Vgl, BMP-5, BMP-6, Vgr-1, and GDF-1. SEQ ID NO: 5 includes all of SEQ ID NO: 4, and also includes at its N-terminus the five amino acid sequence of SEQ ID NO: 8. The generic sequences include both the amino acid identity shared by these sequences in the C-terminal domain, defined by the six- and seven cysteine skeletons (SEQ ID NOS: 4 and 5, respectively), and alternative amino acids for variable positions within the sequence. Positions that allow for alternative amino acids are represented by "Xaa". FIG. 2 shows the alternative amino acids for each "Xaa" position in SEQ ID NOS: 4, 5 and 8. For example, referring to SEQ ID NO: 4 and FIG. 2A, the "Xaa" at position 2 may be a tyrosine or a lysine. The generic sequences provide an appropriate cysteine skeleton for inter- or intramolecular disulfide bonding, and contain certain critical amino acids likely to influence the tertiary structure of the proteins. In addition, the "Xaa," at position 36 in SEQ ID NO: 4, or at position 41 in SEQ ID NO: 5, may be an additional cysteine, thereby encompassing the morphogenically-active sequences of OP-2 and OP-3.

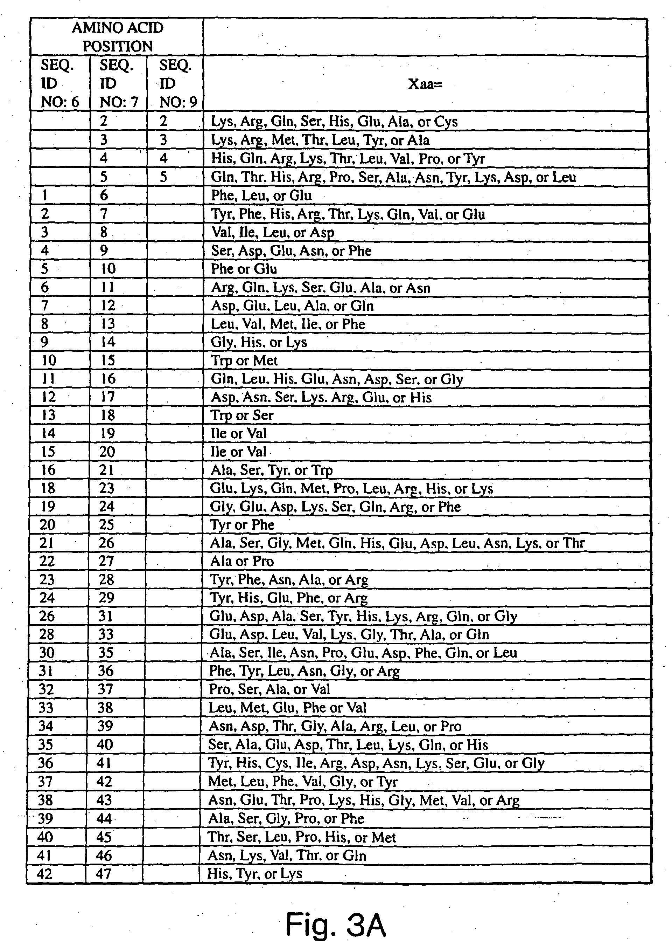

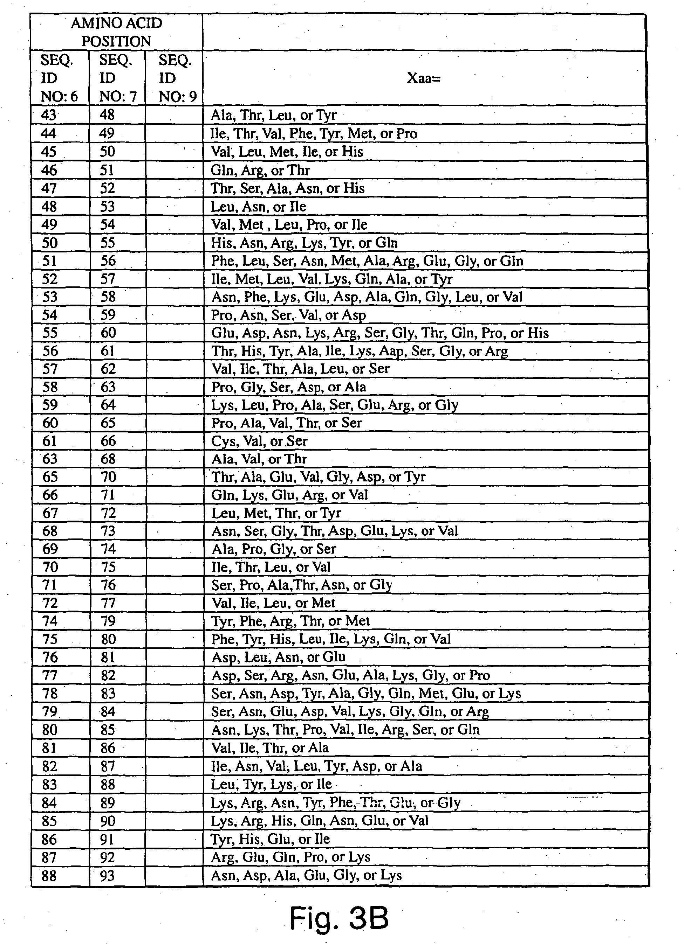

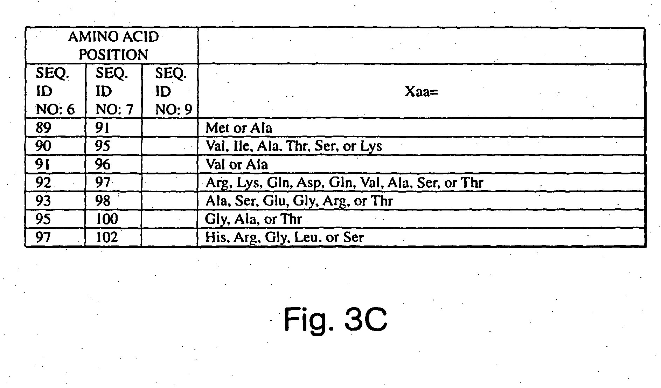

In another embodiment, useful morphogens include those defined by SEQ ID NOS: 6 or 7, which are composite amino acid sequences of the following morphogens: human OP-1, human OP-2, human OP-3, human BMP-2, human BMP-3, human BMP-4, human BMP-5,human BMP-6, human BMP-8, human BMP-9, human BMP-10, human BMP-11, *Drosophila* 60A, *Xenopus* Vg-1, sea urchin UNIVIN, human CDMP-1 (mouse GDF-5, MP52), human CDMP-2 (mouse GDF-6, human BMP-13), human CDMP-3 (mouse GDF-7, human BMP-12), mouse GDF-3, human GDF-1, mouse GDF-1, chicken DORSALIN, *Drosophila* dpp, *Drosophila* SCREW, mouse NODAL, mouse GDF-8, human GDF-8, mouse GDF-9, mouse GDF-10, human GDF-11, mouse GDF-11, human BMP-15, and rat BMP-3b. SEQ ID NO: 7 includes all of SEQ ID NO: 6 and also includes at its N-terminus the five amino acid sequence of SEQ ID NO: 9. SEQ ID NO: 6 accommodates the C-terminal six-cysteine skeleton, and SEQ ID NO: 7 accommodates the seven-cysteine skeleton. Positions that allow for alternative amino acids are represented by "Xaa". FIGS. 3A-3C shows the alternative amino acids for each "Xaa" position in SEQ ID NOS: 6,7 and 9.

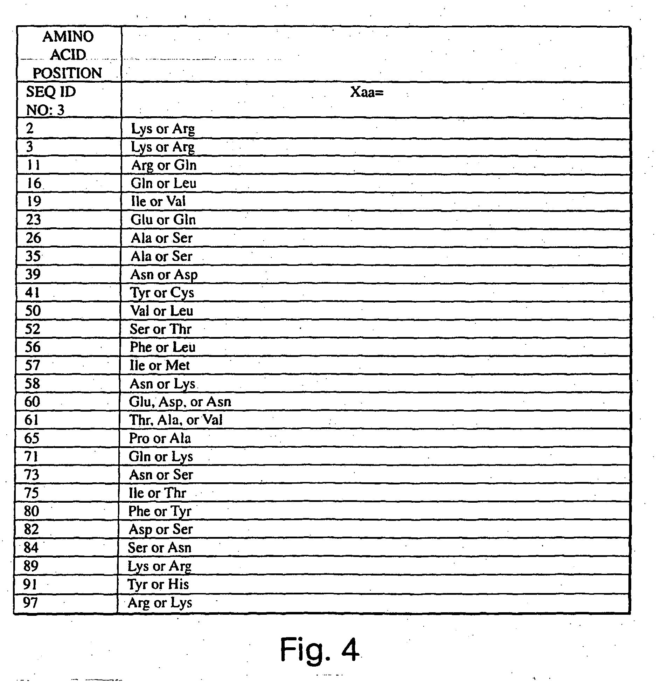

As noted above, certain preferred morphogen sequences useful in this invention have greater than 60% identity, preferably greater than 65% identity, with the amino acid sequence defining the preferred reference sequence of hOP-1. These particularly preferred sequences include allelic and phylogenetic variants of the OP-1 and OP-2 proteins, including the *Drosophila* 60A protein, as well as the closely related proteins BMP-5, BMP-6 and Vgr-1. Accordingly, in certain particularly preferred embodiments, useful morphogens include proteins comprising the generic amino acid sequence SEQ ID NO: 3 (referred to herein as "OPX"), which defines the seven-cysteine skeleton and accommodates the homologies between several identified variants of OP-1 and OP-2. Positions that allow for alternative amino acids are represented by "Xaa". FIG. 4 shows the alternative amino acids for each "Xaa," position in SEQ IDNO: 3. In still another preferred embodiment, useful morphogens include those having an amino acid sequence encoded by a polynucleotide that hybridizes under high stringency conditions with DNA or RNA encoding a reference morphogen. Standard stringency conditions are well-characterized in standard molecular biology texts. See generally MOLECULAR CLONING A LABORATORY MANUAL, (Sambrook *et al*., eds., 1989); DNA CLONING, Vol. I & II (D.N. Glover ed., 1985); OLIGONUCLEOTIDE SYNTHESIS (M.J. Gait ed., 1984); NUCLEIC ACID HYBRIDIZATION (B. D. Hames & S.J. Higgins eds. 1984); B. Perbal, A PRACTICAL GUIDE To MOLECULAR CLONING (1984).

In another embodiment, morphogens useful in the invention include the soluble complex form comprising a mature morphogen dimer linked to a morphogen pro-domain or a solubility-enhancing fragment thereof. A solubility-enhancing fragment is any N-terminal or C-terminal fragment of a morphogen pro-domain that forms a complex with the mature morphogen dimer and increases the solubility of the morphogen dimer. Preferably, the soluble complex comprises a morphogen dimer and two pro-domain peptides. Morphogen soluble complex is described in published application WO 94/03 600, incorporated by reference herein.

In yet another embodiment, useful morphogens include biologically active biosynthetic constructs, including novel biosynthetic morphogens and chimeric proteins designed using sequences from two or more known morphogens. See U.S. Patent No. 5,011,691, incorporated by reference herein (*e*.*g*., COP-1, COP-3, COP-4, COP-5, COP-7, and COP-16).

### II. FORMULATION AND TREATMENT CONSIDERATIONS

As a general matter, compositions and methods of the present invention may be applied to the treatment of any mammalian subject afflicted with cancer. One of ordinary skill in the medical or veterinary arts is trained to recognize whether a mammal is afflicted with cancer. For example, routine testing and/or clinical or veterinary diagnostic evaluation will reveal whether the mammal has any of the symptoms of cancer. Symptoms of cancer include unregulated cell proliferation and/or proliferation of cells that have a cancer-cell phenotype. Other common symptoms of cancer include abnormal bodily function, abnormal cell morphology, abnormal enzyme levels, abnormal hormone levels, abnormal oncofetal antigen levels, abnormal tissue growth, abnormal tissue mass, abnormal tumor-associated protein levels, altered neurologic function, altered neurologic structure, undesirable angiogenesis that provides a tumor with a blood supply, abnormal bleeding, diarrhea, effusions, fatigue, fever, lesions, malnutrition, metastasis, nausea, obstruction of a bodily passageway, opportunistic infection, pain, poor Karnofsky performance status, presence of cancer-associated cell-surface markers, presence of cancer-associated histological markers, presence of cancer-associated intracellular markers, presence of cancer-associated molecular markers, tumor invasion, urinary frequency, and weight loss. See generally, HARRISON'S PRINCIPLES OF INTERNAL MEDICINE 493-627 (Fauci *et al*. eds., 14th ed. 1998).

Cancer can affect any tissue in the body. However, as described above, some tissue sites are more likely to be affected with cancer. Some of the more common cancers that compositions and methods of the present invention are anticipated to treat include adrenal cancer, anal cancer, bladder cancer, bone cancer, brain cancer, breast cancer, cervix cancer, colon cancer, corpus cancer, endocrine cancer, esophageal cancer, fallopian tube cancer, fat cell cancer, gall bladder cancer, gastrointestinal tract cancer, germ cell tumors, kidney cancer, leukemia, liver cancer, lymphoma, lung cancer, muscle cancer, nervous system cancer, ocular tissue cancer, oral cancer, ovarian cancer, pancreatic cancer, prostate cancer, rectal cancer, skin cancer, small intestine cancer, soft tissue cancer, stomach cancer, teratocaremoma (teratomas), testicular cancer, thyroid cancer, ureteral cancer, urinary cancer, uterine cancer, and metastatic cancer of unknown primary site. See generally, HARRISON'S PRINCIPLES OF INTERNAL MEDICINE 493-627 (Fauci *et al*. eds., 14th ed. 1998).

Cancer cell growth is typified by a general unresponsiveness to extracellular signals. However, in accordance with the present invention, it has now been discovered that morphogens can regulate cancer cell growth. As discussed above, BMPRs mediate BMP signaling. For example, in prostate cancer, it has been shown that BMP-2 decreases the rate of proliferation of LNCaP cells (*see* ten Dikje *et al., J. Biol. Chem. 269*: 16985-988 (1994)). It has also been demonstrated that the prostate expresses very high levels of BMPR-IB (*see* Ide *et al*., *Oncogene 14*:1377-382 (1997); Autzen *et al., Br. J. Cancer 78*: 1219-223 (1998)). In accordance with the present invention, it has now been shown that BMP-6 inhibits the growth of murine prostate tumor xenografts expressing all three types of BMPRs (*see* Example 7, *infra*). Hence, the determination of BMPRs expression on targeted malignant cells represents a novel means to pre-screen a subject to enable the selection of subjects with high probability of responding to a given morphogen. Targeted cells may be obtained from a patient, and the expression of specific BMPRs determined, for example, by messenger RNA isolation and Northern Blot analysis. An appropriate morphogen may then be selected which is most likely to provide an effective treatment based upon the BMPR expression in the targeted cells. Alternatively, patients with a low probability of responding to morphogen treatment based upon the absence of requisite BMPR expression on targeted tissue may be referred for alternative therapies.

Morphogens or morphogens combined with one or more other agents in the present invention may be administered by any compatible route. Any suitable means, such as, direct (*e*.*g*., locally, as by injection, implantation or topical administration to a tissue locus), systemic (*e*.*g*., parenterally or orally), external (*e.g*., an i.v. bag), or internal (*e.g*., a bioerodable implant, or a colony of implanted, morphogen-producing cells) can accomplish morphogen delivery capable of inhibiting cancer cell proliferation and/or stimulating differentiation of cells having a cancer cell phenotype. In a preferred embodiment, direct injection of morphogen into tumor tissue results in the necrosis of the tumor and diffuse inflammation. The agent preferably comprises part of an aqueous or physiologically compatible fluid suspension or solution for parenteral administration such as intravenous, subcutaneous, intramolecular, ophthalmic, intraperitoneal, intramuscular, buccal, rectal, vaginal, intraorbital, intracerebral, intracranial, intraspinal, intraventricular, intrathecal, intracisternal, intracapsular, intranasal, or aerosol administration. The morphogen carrier or vehicle is physiologically acceptable so that in addition to delivery of the desired agent to the patient, it does not otherwise adversely affect the patient's electrolyte and/or volume balance. The fluid medium for the agent thus can comprise normal physiologic saline (*e.g*., 9.85% aqueous NaCl, 0.15M, pH 7-7.4). Those skilled in the art can prepare useful solutions for any type of parenteral administration as described, for example, in REMINGTON'S PHARMACEUTICAL SCIENCES (Gennaro, A., ed., Mack Pub., 1990). Dispersing the morphogen, for example, with a dermatologically acceptable carrier such as a lotion, cream, ointment or soap is an appropriate formulation for topical administration to the skin surface and is particularly useful for treating skin cancer.

Association of the mature morphogen dimer with a morphogen pro-domain results in the pro-form of the morphogen that typically is more soluble in physiological solutions than the corresponding mature form. In fact, endogenous morphogens are thought to be transported (*e*.*g*., secreted and circulated) in the mammalian body in this form. Culture medium of morphogen-secreting mammalian cells (*e.g*., cells that are transfected with nucleic acid encoding the morphogen and that are competent to express the morphogen) is one source of this soluble form of the protein. Alternatively, complexing the mature, morphogenically-active polypeptide dimer (or an active fragment thereof) with a morphogen pro-domain polypeptide or a solubility-enhancing fragment thereof creates a soluble species. Solubility-enhancing pro-domain fragments can be any N-terminal, C-terminal, or internal fragment of the pro region of a member of the morphogen family that complexes with the mature polypeptide dimer to enhance stability and/or dissolubility of the resulting noncovalent or covalent complex. Typically, those fragments cleaved at the proteolytic site Arg-Xaa-Xaa-Arg are useful. A detailed description of soluble complex forms of morphogenic proteins, including how to make, test and use them, is described in WO 94/03600 (PCT US 93/07189). In the case of OP-1, useful pro-domain polypeptide fragments include the intact pro-domain polypeptide (residues 3 0-292) and fragments 48-292 and 15 8-292, all of SEQ ID NO: 2. Additionally, other molecules enhance solubility and are particularly useful for oral administrations. For example, addition of 0.2%casein increases solubility of the mature active form of OP-1 by 80%. Other components found in milk and/or various serum proteins may also be useful.

Morphogens, also, may be administered orally. Oral administration of proteins generally is unsuccessful, because digestive enzymes and acids in the mammalian digestive system degrade most proteins before they are absorbed into the bloodstream. However, unlike most proteins, morphogens are typically acid stable and protease-resistant. See, *e*.*g*., U.S. Patent No. 4,968,590. In addition, mammary gland extract, colostrum, and 57-day milk contain at least one morphogen, OP-1. The OP-1 purified from mammary gland extract is morphogenically-active and is detected in the bloodstream. Thus, maternal administration, via ingested milk, may be a natural delivery route of OP-1 and other morphogens proteins. See *e.g*., Letterio *et al*., *Science 264*: 1936-1938 (1994). Moreover, the morphogen found in milk (and mammary gland extract and colostrum) is readily soluble, probably by association with a pro-domain and/or another milk component.

Alternatively or in addition, a delivery vehicle competent for transport through the gastrointestinal system is useful for orally administered morphogens. For example, the morphogen can be a part of a biologically erodible microsphere that interacts with the gastrointestinal lining and absorb through this lining. Useful polymers include, for example, polystyrene, polyactide and/or polyglycolide and/or polycyanoacrylate polymers and combinations thereof.

Morphogens of the present invention must overcome the blood-brain barrier, the brain capillary wall structure that prevents many substances from entering the central nervous system (CNS), in CNS applications. Direct infusion of morphogens into the CNS by methods well known in the art is one manner to bypass the blood-brain barrier. Alternatively, modified morphogens may exhibit enhanced transport across the blood-brain barrier. For example, truncated forms of morphogens may be most successful. Alternatively, those skilled in the art can modify morphogens of the present invention to be derivatized or conjugated to a lipophilic moiety or to a substance that is actively transported across the blood-brain barrier. See, *e*.*g*., Pardridge, *Endocrine Reviews 7*:314-330 (1986) and U.S. Patent No. 4,801,575.

Association of molecules capable of targeting morphogens to a desired tissue with morphogens of the present invention is possible. For example, an antibody, antibody fragment, or other binding protein that interacts specifically with a surface molecule on cells of the desired tissue, is appropriate to target morphogens. The single chain binding site technology disclosed in U.S. Patent No. 5,091,513 is useful to design targeting molecules.

*In vitro, in vivo*, or *ex vivo* applications are useful to expose affected tissues to morphogens. In addition to exposing affected tissues to morphogens, cells capable of expressing morphogens can be delivered to the affected tissue. Those skilled in the art can construct such cells capable of expressing morphogens using standard means. Biocompatible, biodegradable, bioerodable matrices such as collagen or hydroxyapatite are useful to permit cells to infiltrate and grow into the matrix material during differentiation. Moreover, one may remove affected tissues from the host, expose them to any of the variety of morphogens described herein, and then return them to the host. Any of these *in vitro, in vivo,* or *ex vivo* applications are useful to inhibit cancer cell proliferation and/or stimulate differentiation of cells having a cancer cell phenotype away from the cancer cell phenotype. In one aspect of the invention, the growth of NG108-15 cells, embryo carcinoma NTERA-Z CL.D1 cells, LNCaP cells, MCF-7 cells, osteosarcoma cells, human lung carcinoma A-549 cells, and glioblastoma A-172 cells is inhibited.

As will be appreciated by one of ordinary skill in the art, the formulated compositions contain therapeutically effective amounts of the morphogen. The effective dose and dosage strategy will vary depending upon a number of factors including the biological efficacy of the selected agent, the chemical characteristics (*e.g*., hydrophobicity) of the specific agent, the formulation of the agent, the administration route, the target location, the pharmacokinetics of the agent, the condition of the diseased tissue, and the overall health status of the particular mammal. For example, intratumoral administration of about 200 µL of 100 ng/mL BMP-6 inhibited tumor growth in mice (*see* Example 7, *infra*).

Morphogens of the invention may be administered alone or in combination with other molecules known to be beneficial in the treatment of cancer. For example, one can administer various well-known anti-tumor agents, analgesics, or anti-inflammatory compounds with a morphogen. Additionally, one can administer various well-known growth factors, hormones, enzymes, therapeutic compositions, antibiotics, or other bioactive agents with a morphogen.

### III. EXAMPLES

The following examples provide experimental evidence of the effect of various morphogens on cell differentiation and cell growth in various cell types. Each of these cancer cell lines is characterized by some form of unregulated growth. As shown below in a variety of examples, the cell growth of cancer cells exposed to morphogens was inhibited. Moreover, in several of the examples, morphological characteristics of cell differentiation were observed. These experimental data provide positive evidence that morphogens can alleviate the symptoms of cancer, possibly by inhibiting cell growth and/or stimulating cell differentiation of cancer cells.

### Example 1 Morphogen-Induced Cell Differentiation of NG108-15 Neuroblastoma/Glioma Cell Line

As one example of morphogen-induced cell differentiation, the effect of OP-1 on the differentiation of neuronal cells was tested in culture. Specifically, the effect of OP-1 on the NG108-15 neuroblastoma x glioma hybrid clonal cell line was assessed. The NG108-15 cell line (also referred to as simply "NG 108") displays a fibroblastic-type morphology in culture. The cell line can be induced to differentiate chemically using 0.5 mM butyrate, 1% DM50 or 500 mM Forskolin, inducing the expression of virtually all important neuronal properties of cultured primary neurons. However, chemical induction of these cells also induces cessation of cell division.

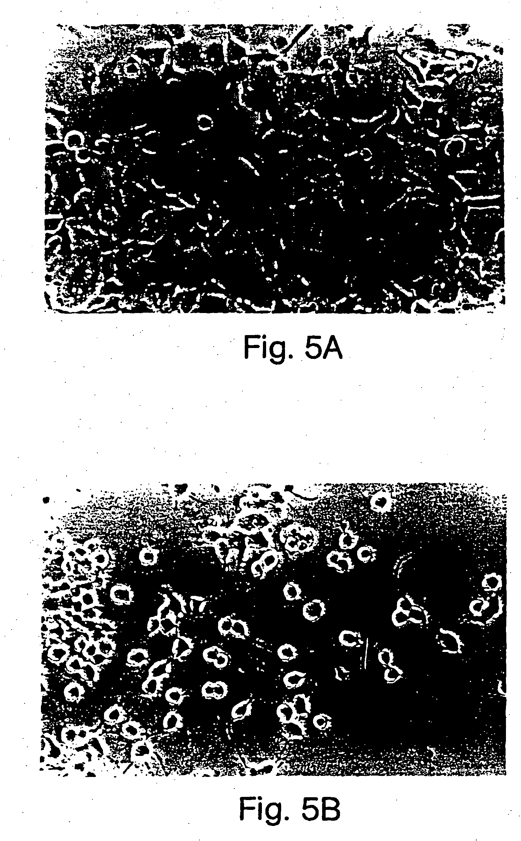

In the present experiment NG108-15 cells were subcultured on poly-L-lysine coated 6-well plates. Each well contained 40,000-50,000 cells in 2.5 ml of chemically defined medium. This chemically defined medium contained Dulbecco's modified Eagle's medium: Ham's F- 12 (3:1), NaHCO₃ (3.7 g/liter), trace elements (0.5 nM Mo, 0.5 nM Mn, 5 nM Cd, 15 nM Se, 25 nM Sn, 0.25 nM Ni, and 0.25 nM Si), 25 mM HEPES, 10 :g oleic acid complexed to fatty acid-free albumin, 50 µg/ml transferrin, 25 µg/ml insulin, 2 mM glutamine, and 25 nM Na₃VO₄. See Chamess *et al., J. Biol. Chem. 261*: 3164-3169 (1986); Perides *et al., Proc. Natl. Acad. Sci. USA 89*: 10326-30 (1992), incorporated herein by reference. On the third day, 2.5 :1 of OP-1 in 60% ethanol containing 0.025% trifluoroacetic was added to each well. OP-1 concentrations of 0, 1, 10, 40 and 100 ng/ml were tested. The media was changed daily with new aliquots of OP-1. After four days with 40 and 100 ng OP-1/ml concentrations, OP-1 induced differentiation of NG108 cells. FIG. 5 shows the morphological changes that occur. The OP-1 induced clumping and rounding of the cells and the production of neurite outgrowths characteristic of the neuronal phenotype (compare FIG. 5A (naive NG 108 cells) with FIG. 5B, showing the effects of OP-1 treated cells). Thus, the OP-1 can induce the cells to differentiate into a neuronal cell morphology. Some of the outgrowths appeared to join in a synaptic-type junction. This effect was not seen in cells incubated with TGF-β1 at concentrations of 1 to 100 ng/ml. The neuroprotective effects of OP-1 were demonstrated by comparison with chemical differentiation agents on the NG108 cells. 50,000 cells were plated on 6-well plates and treated with butyrate, DMSO, Forskolin or OP-1 for four days. Cell counts demonstrated that in the cultures containing the chemical agents the differentiation was accompanied by a cessation of cell division. In contrast, the cells induced to differentiate by OP-1 continued to divide, as determined by [³H]-thymidine uptake. The data suggest that OP-1 is capable of maintaining the stability of the cells in culture after differentiation.

### Example 2 Morphogen-Induced Differentiation and Inhibition of Proliferation of a Human Teratocarcinoma Cell Line

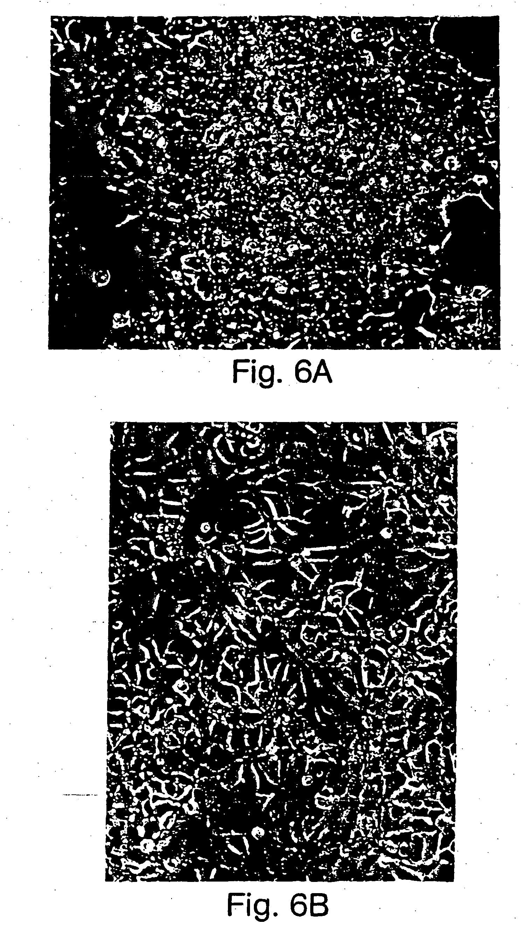

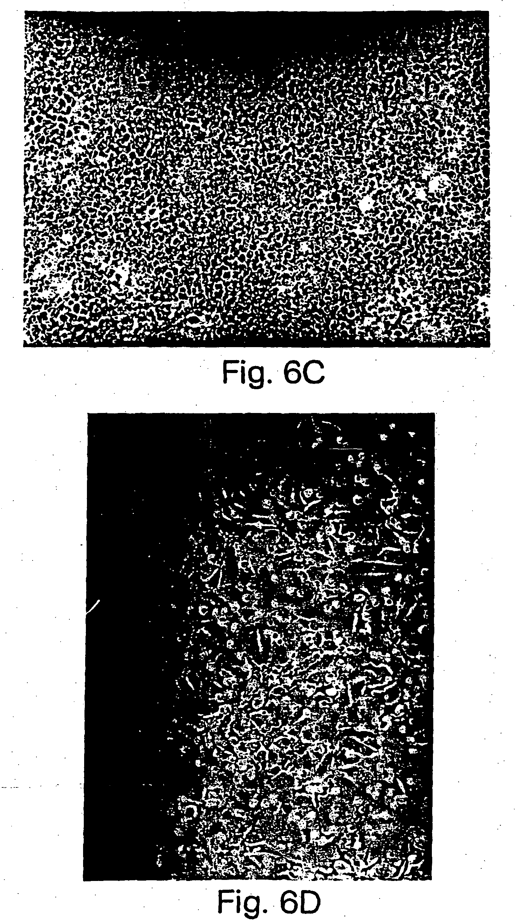

Another related example demonstrates the ability of the morphogens of the invention to induce the "redifferentiation" of transformed cells. The human embryonal carcinoma (EC) cell line NTERA-2CL.D1 ("NTERA2") is derived from a human teratocarcinoma. *See* Andrews *et al. Lab. Invest. 50*: 147-167 (1984). In the absence of an external stimulant these cells can be maintained as undifferentiated stem cells, and can be induced to grow in serum free media(SFM). In the absence of morphogen treatment, the cells proliferate rampantly and are anchorage-independent. The effect of morphogen treatment is seen in FIGS. 6A-D. FIGS. 6A and 6B show 4 days of growth in SFM in the presence of OP-1 (25 ng/ml, 6A) or the absence of morphogen (6B). FIGS. 6C and 6D are 5 days growth in the presence of 10 ng/ml OP-1 (6C) or no morphogen (6D). FIGS. 6C and 6D are at 10x and 20x magnification compared to FIGS. 6A and 6B. As can readily be seen, in the presence of OP-1, the EC cells grew as flattened cells, becoming anchorage dependent. In addition, growth rate was reduced approximately 10-fold. Finally, the cells were induced to differentiate. These results indicate that in a teratocarcinoma cell line, morphogens differentiate and inhibit growth of the cells.

### Example 3 Morphogen Inhibition of Proliferation of the Androgen-Responsive Prostate Cancer Cell Line LNCaP

Approximately 25% of all males over the age of 55 years old are afflicted by some form of prostate disease. Androgens are known to stimulate prostate cancer cell proliferation. Typically, prostate cancer cells eventually lose their dependence on androgen to proliferate and the cancer progresses to a more malignant, androgen-independent state. Loss of androgen responsiveness is also a characteristic of metastatic forms of prostate cancer. Thus, a common method of treating prostate cancer is to employ some form of androgen control or ablation to prevent androgen-stimulated proliferation and an eventual progression to a more malignant and metastatic form of prostate cancer.

Early detection of prostate cancer has more recently become possible with the development of sensitive tests for circulating antigens that are diagnostic for prostate cancer. Such antigens include prostate serum antigen (PSA), prostatic acid phosphatase (PAP), and prostatic inhibin (PIP). Early detection affords the opportunity for early treatment. Currently, the most common available methods of treating prostate cancer include surgery or radiotherapy to eliminate the cancerous prostate gland. In addition, various methods of hormone therapy (*e*.*g*., androgen ablation or control) are commonly employed to slow the development of solid and metastatic prostate cancer. However, patients subjected to these currently available therapies for treating prostate cancer are also at risk for various undesirable side effects, notably, incontinence and impotence. Thus, there is a need for a therapy that is not associated with such undesirable side effects.

Schneyer and co-workers previously studied the effect of the protein activin, a non-morphogen member of the TGF-β superfamily, as a therapeutic agent to treat prostate cancer. See, *e*.*g*., Wang *et al., Endocrinology 137*: 5476-5483 (1996). However, high renal toxicity has thwarted further development of activin as an effective therapeutic anti-cancer agent. As described herein, it has now been found that morphogens, such as OP-1 and BMP-6, are capable of inhibiting proliferation of both androgen-dependent and androgen-independent prostate cancer cells. In contrast to activin, morphogens such as OP-1 and BMP-6 are known to be compatible with bone tissue, kidney function, and other soft tissues of the body. Accordingly, this invention provides a method of treating prostate cancer comprising administering a morphogen, such as OP-1 or BMP-6, to a prostate cancer patient. Once a morphogen has been administered to a patient, morphogen-mediated inhibition of prostate cancer cell proliferation may be monitored by a clinician using various standard methods, including examination to detect a decrease in tissue size and texture, measuring of the levels of circulating antigens, such as PSA, and monitoring of patient discomfort and genito-urinary tract function.

As shown herein, morphogens, such as BMP-6 and OP-1, inhibit proliferation of prostate cancer cells as demonstrated using the androgen-responsive LNCaP cell line, an established *in vitro* model of androgen-responsive prostate cancer. The LNCaP prostate cancer model was employed in three experiments. First, the effect of BMP-6, OP-1 (BMP-7), Activin, or TGF-β1 on cell growth of LNCaP cells in the absence of exogenous androgen was examined. Second, the effect of BMP-6, OP-1 (BMP-7), Activin, or TGF-β1 on cell growth of LNCaP cells exposed to androgen was examined. Third, the effect of follistatin, an activin and morphogen binding protein, on the bioactivity of BMP-6, OP-1 (BMP-7), and Activin in LNCaP cells was examined.

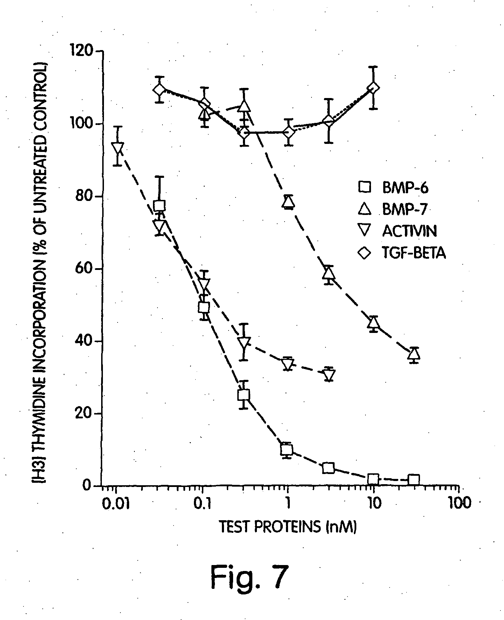

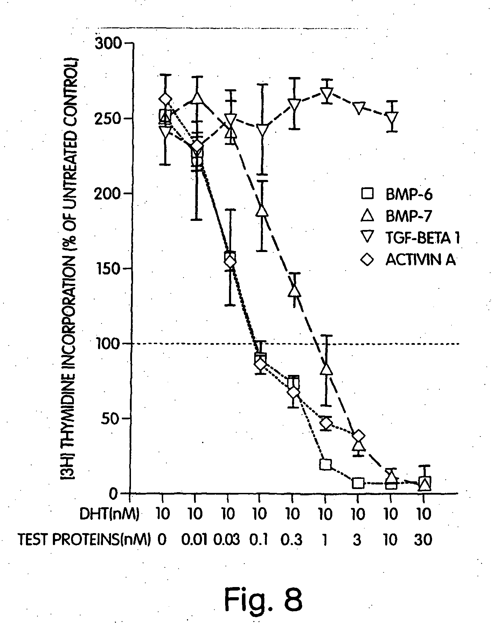

LNCaP cells were obtained from the American Type Culture Collection (Rockville, MD) and grown in 75 cm flasks with RPMI-1640 containing 10% fetal bovine serum (RPMI/10%FBS) in a humidified atmosphere of 5% CO₂:95% air. Culture medium was changed every third day. For assay of activin and BMPs the stock cultures were subcultured by replating trypsinized cell suspensions onto 96 well plates. For this purpose, LNCaP cells were plated in 24 well plates at 3x10⁴ cells/well in 0.5 mL of RPMI/10% FBS, and incubated for 48 hours to establish subconfluent cultures for study. After the preincubation period, the medium was gently aspirated, the cells were washed once with warm RPML, and the cells were subsequently cultured in 0.5 mL of RPMI containing 2% FBS for the study of treatment effects on cell proliferation rate (*e*.*g*., cell growth). Varying doses of activin, BMPs, DHT, or FS, alone or in combination, were added to triplicate wells and incubations were continued for another 48 hours. After [³H]-thymidine incorporation into cells, the cells were washed once with RPMI and precipitated with 0.5 mL of 10% cold trichloroacetic acid (TCA) for 20 minutes at 4°C. After two methanol washes, the precipitate was solubilized in 0.5 mL of 1 N sodium hydroxide and the amount of radioactivity was counted in a liquid scintillation counter. [³H]-thymidine incorporation is expressed as cpm/well and represents a quantitative measurement of cell proliferation rate. In the first experimental paradigm, LNCaP cells (in the absence of exogenous androgen) were exposed to various concentrations of BMP-6 OP-1 (BMP-7), Activin, or TGF-β1 as indicated. The effect of these substances on LNCaP cells was assessed using a [³H]-thymidine uptake assay. FIG. 7 shows that activin and the morphogens, BMP-6 and OP-1 (BMP-7), inhibit cell growth in a dose dependent manner. BMP-6 had a higher potency than OP-1 (BMP7). In the second experimental paradigm, LNCaP cells (exposed to 10 nM DHT androgen) were exposed to various concentrations of BMP-6, OP-1 (BMP-7), Activin or TGF-β1 in the presence of androgen (10 nM DHT) and the effect of these substances, was assessed using [³H]-thymidine uptake assay. FIG. 8 shows that activin and the morphogens (BMP-6 and OP-1 (BMP-7), inhibited proliferation of the androgen-stimulated LNCaP cells in a dose dependent manner. BMP-6 had a higher potency than OP-1 (BMP-7).

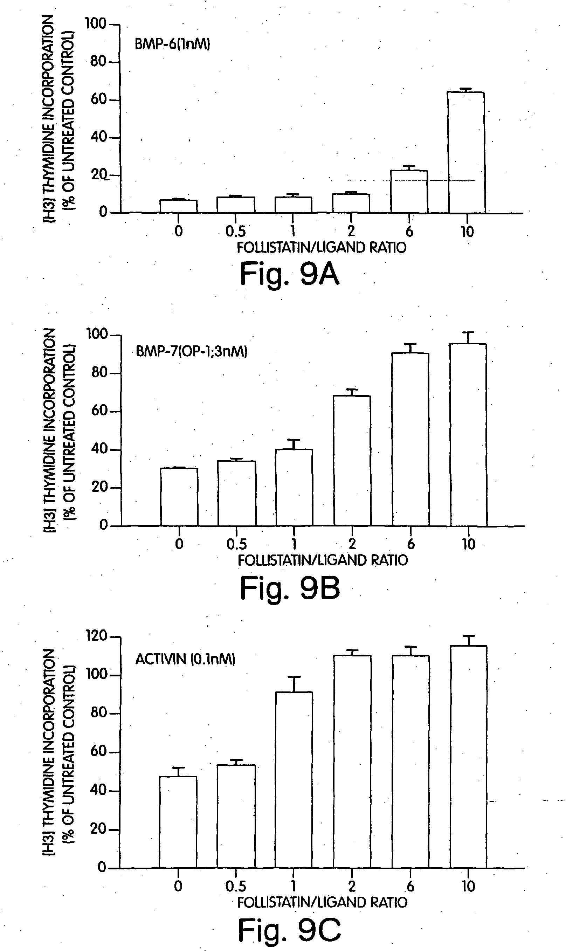

In the third experimental paradigm, LNCaP cells were exposed to 1 nM BMP-6, 3 nM OP-1 (BMP-7), or 0.1 nM Activin and subsequently were exposed to the activin/BMP-binding protein follistatin (FS) in a ratio of follistatin: ligand of 0.5:1, 1:1, 2:1, 5:1, and 10:1. The effects of follistatin and ligand on proliferation of the LNCaP cells were assessed using [³H]-thymidine uptake assay. FIG. 9 shows that follistatin neutralized the bioactivity of both morphogens, BMP-6 and OP-1 (BMP-7).

### Example 4 Morphogen Inhibition of Proliferation and Induction of Differentiation of 5 Estrogen Responsive Human Breast Cancer MCF-7 Cells

This example demonstrates that the morphogen, OP-1, inhibits growth of estrogen responsive human breast cancer MCF-7 cells. MCF-7 cells were grown in monolayer cultures in Eagle's MEM supplemented with 5% fetal bovine serum (FBS), 2 mM L-glutamine, Earle's BSS, 1 mM sodium pyruvate, antibiotics, and 10 mg/ml bovine insulin. To prevent possible steroid-like artifacts from phenol red containing medium, before experimental treatments, the cells were incubated with phenol-free, serum-free medium or phenol-free medium supplemented with 5% FBS, each stripped of endogenous steroids with dextran-coated charcoal.

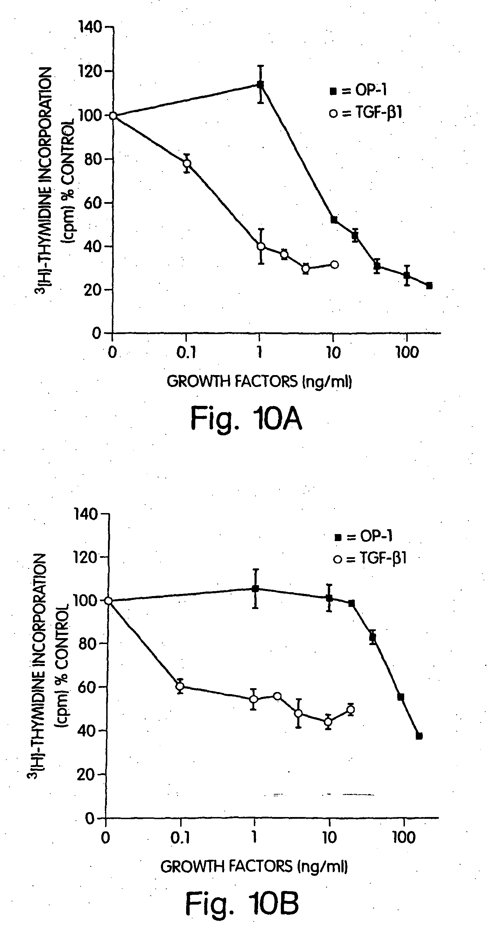

In the first experiment, the effect of OP-1 and TGF-β on proliferation of MCF-7 cancer cells was tested in the presence and absence of serum. The cells were grown in 24- or 48-well plates. OP-1 or TGF-β1 was added at various concentrations, as indicated, in serum free medium, or, alternatively, OP-1 or TGF-β1 was added in 5% serum containing medium at various concentrations, as indicated. FIGS. 10A and 10B show that both OP-1 and TGF-β1 inhibited cell growth in a dose dependent manner using a [³H]-thymidine uptake assay. In the presence or absence of serum, OP-1 inhibited cell growth by approximately 80% of control at 200 ng/ml (FIG. 10A, filled squares) and by approximately 65% of the control at 200 ng/ml in the presence of 5% serum containing medium (FIG. 10B, filled squares). TGF-β1 inhibited cell growth by approximately 65% of control at 10 ng/ml in serum free medium (FIG. 10A, unfilled circles) and by approximately 55% of control at 10 ng/ml in 5% serum containing medium (FIG. 10B, unfilled circles).

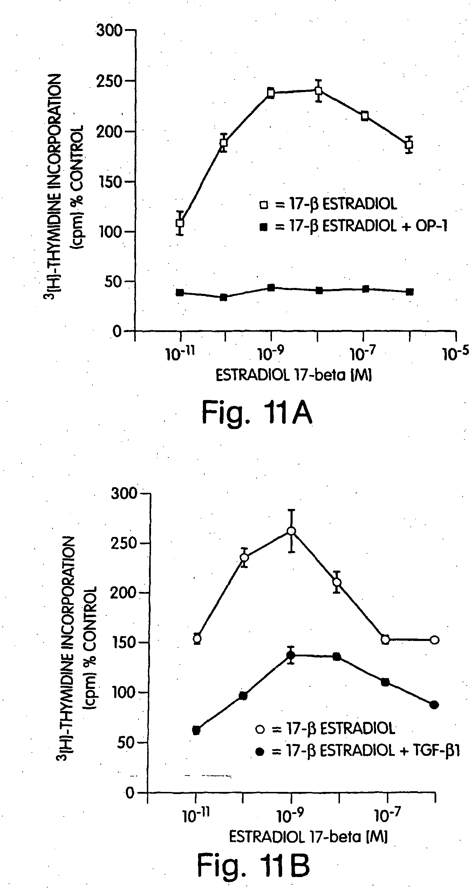

In a related experiment, the effects of OP-1 or TGF-β1 on 17-β estradiol-stimulated MCF-7 breast cancer cells were examined. The MCF-7 cells were incubated in medium supplemented with 17-β estradiol (1 x 10⁻¹¹, 10⁻¹⁰, 10⁻⁹, 10⁻⁸, 10⁻⁷, 10⁻⁶ M) and then exposed to 200 ng/ml of OP-1. The effects on cell growth were assayed using a [³H]-thymidine uptake assay. FIG. 1A shows that while MCF-7 cells exposed to 17-β estradiol and not OP-1 had an increase of cell growth of approximately 250% of control at 10⁻⁹ M 17-β estradiol (unfilled squares). Those MCF-7 cells exposed to both 10⁻⁹ M 17-β estradiol and OP-1 showed an inhibition of cell growth of approximately 80% of control (filled squares). Using the same protocol described immediately above, 10 ng/ml TGF-β1 was substituted for OP-1. FIG. 11B shows that TGF-β1 inhibited cell growth by approximately 40% of control in MCF-7 cells stimulated with 10⁻¹¹ M 17-β estradiol (filled circles).

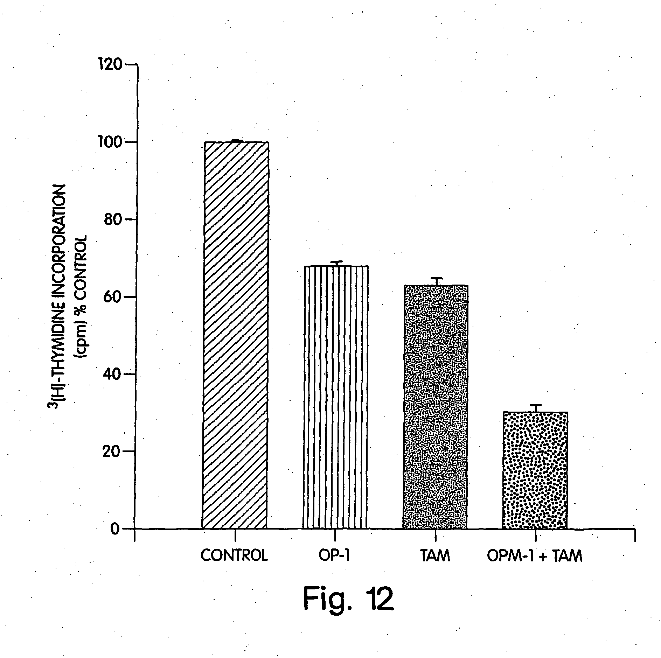

In another experiment, tamoxifen, an estrogen antagonist that is known to inhibit growth of MCF-7 cells, was examined for its effects in comparison to as well as in addition with OP-1. MCF-7 cells were exposed to 1 x 10⁻⁶ M tamoxifen, 100 ng/ml of OP-1, or both tamoxifen and OP-1 combined. Cell growth was assayed using a [³H]-thymidine uptake assay. FIG. 12 shows that OP-1 or tamoxifen at these concentrations inhibited cell growth by approximately 40% of control. Together, OP-1 and tamoxifen inhibited cell growth by approximately 70% of control.

In another related experiment, the OP-1 gene was stably transfected into MCF-7 cells using standard calcium phosphate precipitation. The major immediate early promoter (MIE) of Cytomegalovirus, isolated from plasmid pCDM8 (Invitrogen), was used to direct the transcription of the OP-1 cDNA. Both SV4O early region splice signals and poly-A addition signals obtained from pMAMneo (Clonetech, Inc.) were added as 3' processing elements. A shorter 3' region derived from the same vector, without the intron and splice sites, also has been used with equal success. The eukaryotic selection marker neomycin also was present, under the transcriptional control of SV40 early promoter. See U.S. Patent No. 5,712,119; U.S. Patent No. 5,614,385; U.S. Patent No. 5,585,237, each incorporated herein by reference.

One day prior to transfection, cells were plated at a density of 1 x 10⁶ cells/100 mm petri dish. Fifty micrograms of purified DNA prepared from the plasmid containing the OP-1 gene was transfected into the cells by the calcium phosphate precipitation method. Two days post-transfection, cells were trypsinized and plated 1:3 onto fresh plates containing selection medium containing 400 mg/ml G418. G418 resistant colonies were visible about two weeks later, and some of these cells were used to verify OP-1 expression by Northern Blot analysis.

When MCF-7 cells were transfected with the OP-1 gene, a change in morphology(fibroblast-like) occurred. Cellular responsiveness to estrogen also was altered. The OP-1 transfected MCF-7 cells were treated with various doses of 17-β estradiol for 3 days (10⁻⁷-10⁻¹¹ M 17-β estradiol). Cell growth was assayed using a [³H]-thymidine uptake assay. 17-β estradiol failed to stimulate cell growth. In fact, MCF-7 cells transfected with the OP-1 gene showed a dose-dependent inhibition of cell growth of approximately 50% of control at 1x10⁻⁷ M 17-β estradiol.

These findings collectively demonstrate that OP-1 inhibits both basal and estrogen stimulated growth of MCF-7 breast cancer cells. OP-1 also induces cellular differentiation in MCF-7 cells, possibly a switch from epithelial cell type to mesenchyme, consistent with the use of morphogens such as OP-1 as a therapeutic agent for certain estrogen-responsive human breast cancers.

### Example 5 Morphogen Inhibition of Proliferation of Human Glioblastoma A-172 Cells

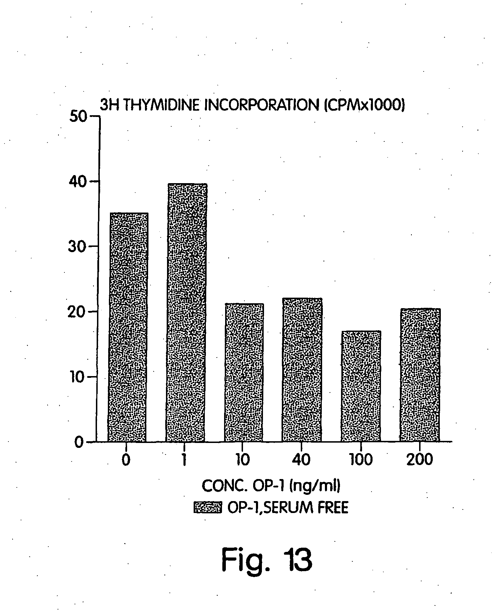

This example demonstrates that the morphogen, OP-1, inhibits cell growth of human glioblastoma A-172 cells. A-172 cells were plated 5 x 10⁴ cells per well in a 48-well plate in DMEM containing 10% FBS. After 24 hours, the growth medium was replaced with serum free DMEM. OP-1 (1, 10, 40, 100, 200 ng/ml) was added to the cells and incubated at 37°C in the presence of 5% CO₂ for 24 hours, which included a 6 hour, pulse with 1 µCi [³H]-thymidine. After the incubation, the cells were washed with PBS. TCA (10%) was added and the cells were incubated for 15 minutes. The cell layer was washed three times with distilled water and was extracted with 1% SDS. The incorporated radioactivity was measured in a liquid scintillation analyzer. FIG. 13 shows that the morphogen, OP-1, inhibited the proliferation of A-172 cancer cells by approximately 50% of control at 100 ng/ml.

### Example 6 Morphogen Inhibition of Proliferation of Human Lung Carcinoma A-549 Cells

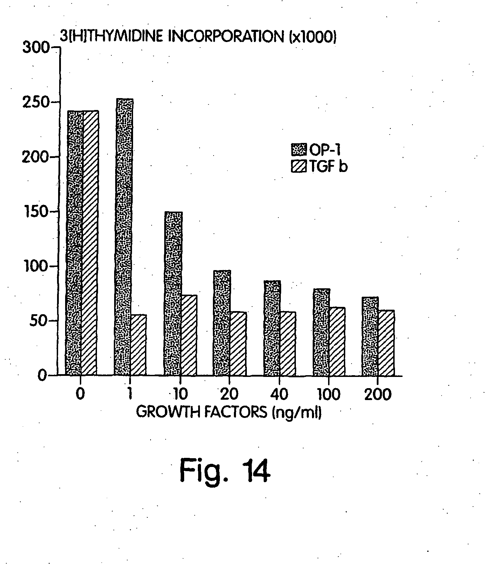

This example demonstrates that the morphogen, OP-1, inhibits cell growth of human lung carcinoma A-549 cells. A-549 cells were plated 5 x 10⁴ cells per well in a 48-well plate in DMEM containing 10% FBS. After 24 hours, the growth medium was replaced with serum free DMEM. OP-1 (1, 10, 20, 40, 100, 200 ng/ml) was added to the cells and incubated at 37°C in the presence of 5% CO₂ for 24 hours, which included a 6 hour pulse with 1 µCi [³H]-thymidine. After the incubation, the cells were washed with PBS. 10% TCA was added and the cells were incubated for 15 minutes: The cell layer was washed three times with distilled water and was extracted with 1% SDS. The incorporated radioactivity was measured in a liquid scintillation analyzer. FIG. 14 shows that OP-1 inhibited the cell growth of A-549 1mg cancer cells in a dose dependent manner with a 70% inhibition achieved at 200 /ml. TGF-132, on the other hand, maximally inhibited 77% of cell growth at 1 ng/ml.

### Example 7 Morphogen-induced Inhibition of Tumor Cell Proliferation in vitro

The effect of morphogen on the proliferation of two murine prostate cancer cell lines was examined *in vitro.* Initially, the expression of BMP receptors (BMPRs) in these mouse prostate cancer cell lines was investigated by Northern blot analysis. The results demonstrated that both cell lines express three types of BMPRs and that a morphogen, BMP6, inhibits the proliferation of these cell lines in a dose-dependent manner.

### Cell Culture

Two cell lines, derived from prostate cancer of TRAMP (Transgenic Adenocarcinoma of Mouse Prostate) mice and designated E4 and F11, were maintained in Dulbecco's Modified Eagle Medium (DMEM) supplemented with 10% Fetal Bovine Serum (FBS) 5 µg/ml inculin, and 10⁸ M dihydroresterone (*see* Foster *et al*., *Cancer Res. 7*: 3325-330 (1997)). All cells were derived from the 20^{th} through 23^{rd} passages.

Prior to treatment with BMP-6, the media were switched to DMEM supplemented with 1% FBS. Then BMP-6 was added at following concentrations: 1, 10, and 100 µg/ml. The media were changed every other day. At the end of a 6-day period, cells were harvested and counted using a hemacytometer. All assays were performed three times and similar results were obtained each time.

### Messenger RNA Isolation and Northern Blot Analysis

The expression of BMPRs in E4 and F11 cells was investigated by Northern blot analysis. After harvesting the cells, mRNA was isolated using a commercial kit (Ribosep, Collaborative Biomolecules, Bedford, MA) according to the manufacturer's recommended protocol. For Northern blot analysis, 2 µg of poly A RNA were separated by electrophoresis in 1% formaldehyde-agarose gel and transferred to nylon membranes (Zeta-Probe GT membrane, Bio-Rad Laboratories, Hercules, CA). The membranes were subsequently rinsed in 2X SSC (1X SSC = 0.15 M Na Citrate, pH 7.0). Then the separated RNA was cross-linked to the nylon membrane using an ultraviolet light cross linker. Prehybridization was performed in 50% formamide, 0.12 M Na₂HPO₄ (pH 7.2), 0.25 M NaCl, 7% (wt/vol) SDS, and 250 µg/ml heat-denatured herring sperm DNA (Promega Corp. Madison, WI) for 2 hrs at 42°C. Hybridization was performed overnight at 42°C in then prehybridization solution containing probe labeled with [³²P]-deoxy-CTP (Amersham Corp., Arlington Heights, IL) using a random oligonucleotide priming kit (Random Primers DNA labeling system, Life Technologies). After washing the membranes in sequential dilutions of SSC, autoradiography was carried out using Kodak X-Moat AR film (Eastman Kodak, Rochester, NY) with an intensifying screen at -70°C.

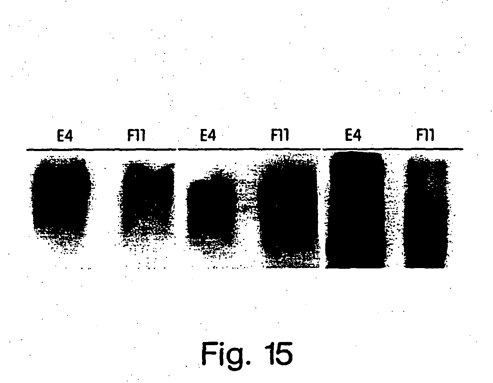

As shown in FIG. 15, both cell lines expressed the three types of BMPRs: BMPR-IA, BMPR-IB, and BMPR-II. As previously reported, there were three splice variants for BMPR-II. *See* Rosenzweig *et al., Proc. Natl. Acad. Sci. USA 92*: 7632-636 (1995).

### Inhibition of Proliferation

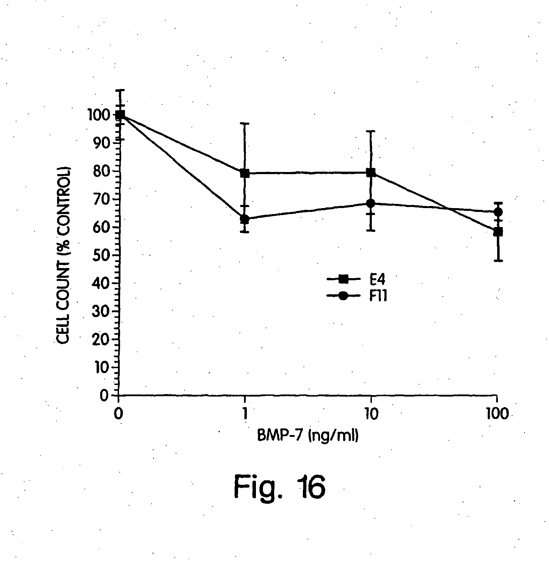

The addition of BMP-6 to these cell lines in tissue culture dramatically decreased the rate of proliferation in a dose-dependent manner (see FIG. 16). Specifically, in the presence of 100 ng/ml BMP-6, the cell count of both E4 and F11 cells were approximately 60% of that of the control (no BMP-6). Since the two cell lines expressed all three types of BMPRs and were sensitive to BMP-6, E4 cell line was chosen randomly for subsequent analysis concerning the therapeutic potential of BMP-6, *in vivo*.

### Example 8 Morphogen-induced Inhibition of Tumor Progression in vivo

The ability of morphogen to inhibit the progression of cancer *in vivo* was tested using the murine prostate cancer cell line E4, as discussed in Example 7. Adult male C57BL/6 mice were purchased and maintained according to the NH standards established in the Guidelines for the Care and Use of Experimental Animals. Thirty mice were inoculated with 10⁷ cells subcutaneously. At 6 weeks, 21 mice had palpable tumor. These animals were divided into three groups of 7 each. The experimental group received 200 µl of 100 ng/ml BMP-6 while the control groups received either the vehicle (20 mM acetate and 5% Mannitol, pH 4.5) or no treatment. Injections were delivered directly to the tumors once every other day for 3 weeks. The animals were observed for 10 weeks after the initiation of therapy, and tumor volume was measured in each animal weekly.

At the end of the ten-week period, all tumors were harvested and preserved in formalin and embedded in paraffin. Subsequently, 5 µm sections were obtained from each tumor and stained with hematoxylin and eosin (H&E).

### Inhibition of tumor progression

At the end of the 10-week period after the initiation of treatment, 5 of 7 mice treated with BMP-6 were alive while all animals in the other group were dead (Table 1). Of the five animals, two had unpalpable tumor.

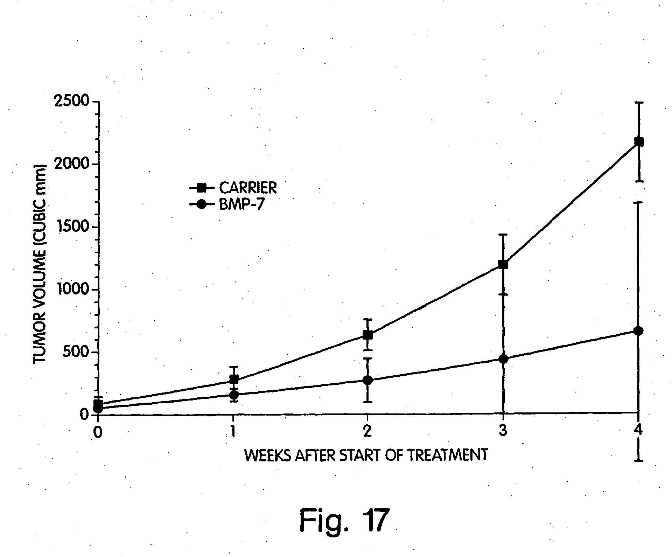

In addition to the rate of survival, BMP-6 treatment also decreased the rate of tumor growth. The results, shown in FIG. 17, demonstrate that the average tumor in mice treated with BMP-6 was approximately one third of that in mice given vehicle only. There was no significant difference between the rate of tumor growth between the group that received the vehicle and the animals that had no treatment (data not shown).

**Table 1.**

| Rate of Survival of Mice Treated with BMP-6 | | |
|---|---|---|
| **Innoculum** | **Survival No./Total No. %** | **P-value** |
| None | 0/7 (0) | |
| Vehicle | 0/7 (0) | |
| BMP-6 | 5/7 (71)** | <0.001 |

| | | |
|---|---|---|
| *Survival at 10 weeks after tumor became palpable. **Two had unpalpable tumor while 2 had necrotic center. | | |

### Data Analysis

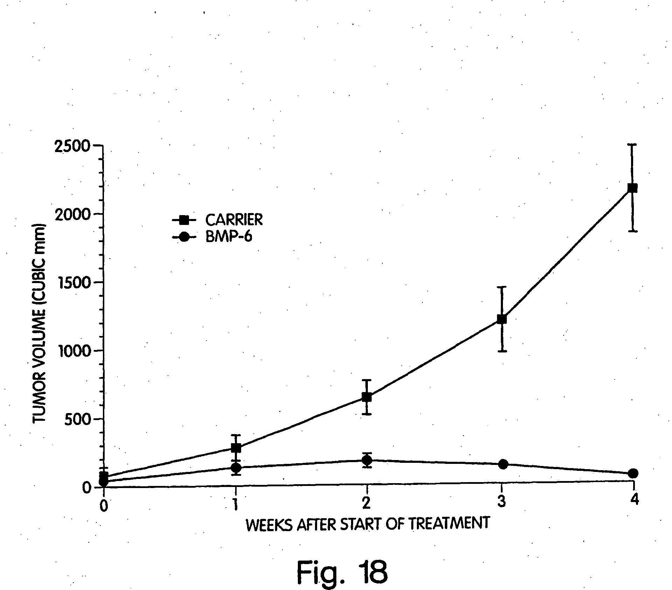

As an attempt to determine the effect of morphogen treatment on mice that responded to the treatment, re-analysis of the data was carried out after removing those from the two non-surviving animals, which did not respond to BMP-6 treatment (FIG. 18). All statistics were evaluated by the χ² test, and P<0.05 was considered statistically significant. The new data analysis indicates that tumor volume actually started to decrease after 2 weeks of BMP-6 injection in animals that responded to the treatment. At the end of 4 weeks after the initiation of BMP-6 administration, the average tumor size of the mice that responded was approximately 5% of that of the control group that received the vehicle only.

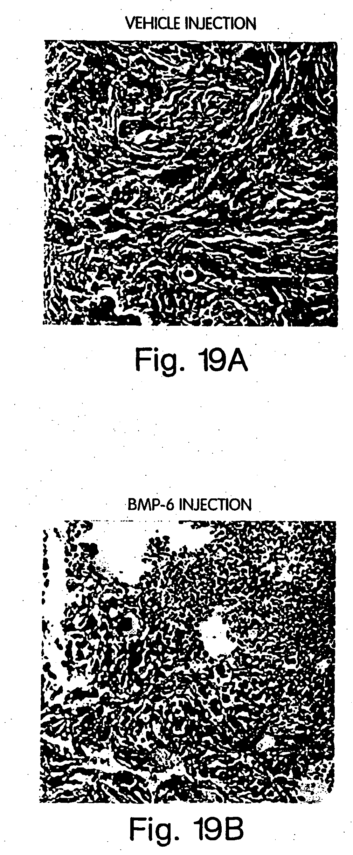

As an initial attempt to determine the mechanism underlying the morphogen-induced tumor regression seen in the present study, the tumors were harvested and stained with H&E for microscopy. As shown in FIGS. 19A and 19B, tumors that were treated with BMP-6 showed severe necrosis and diffuse inflammation.

### OTHER EMBODIMENTS

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

Other embodiments of the invention are within the following claims.

## Claims

1. Use of a morphogen for the manufacture of a medicament for alleviating cancer symptoms, wherein the morphogen inhibits cancer cell growth and is selected from the group consisting of: BMP-9, BMP-11, BMP-14, BMP-15, GDF-8, GDF-9, GDF-11, ADMP and NEURAL.

2. Use according to Claim 1, wherein the cancer cells are NG108-15 cells, LNCaP cells, MCF-7 cells, osteosarcoma cells, human lung carcinoma A-549 cells, TRAMP cells, NTERA2 cells, or glioblastoma A-172 cells.

3. Use according to Claim 1, wherein said cancer is bone cancer, brain cancer, breast cancer, germ cell tumors, lung cancer, nervous system cancer, prostate cancer, or teratocarcinoma.

4. Use according to Claim 1, wherein the morphogen is non-covalently associated with at least one polypeptide, where the polypeptide is a prodomain of OP-1, OP-2, 60A, GDF-1, BMP-2A, BMP-2B, DPP, Vg1, Vgr1, BMP-3, BMP-5, or BMP-6.

5. Use according to Claim 4, wherein the morphogen is non-covalently associated with more than one pro-domain polypeptide.

6. Use according to Claim 1, wherein the morphogen is present in a pharmaceutically acceptable vehicle.

7. Use according to Claim 6, wherein the vehicle is selected from the group consisting of biocompatible microspheres, biodegradable microspheres, and bioerodable microspheres.

8. Use according to Claim 6, wherein the vehicle is selected from the group consisting of biocompatible matrices, biodegradable matrices, and bioerodable matrices.

9. Use according to Claim 6, wherein the vehicle is an aqueous solution.

10. Use according to Claim 1, wherein the medicament comprises cells capable of expressing the morphogen.

11. Use according to Claim 1, wherein the medicament comprises a non-infectious, non-integrating DNA encoding the morphogen operably linked to a promoter.

12. Use according to Claim 11, wherein the DNA is free from association with transfection-facilitating proteins, viral particles, liposomal formulations, charged lipids, and calcium phosphate precipitating agents.

13. Use according to Claim 11, wherein the non-infectious, non-integrating DNA encoding the morphogen operably linked to a promoter further comprises an enhancer element in operative association with the promoter.

14. Use according to Claim 1, wherein the cancer is selected from the group consisting of adrenal cancer, anus cancer, bladder cancer, bone cancer, brain cancer, breast cancer, cervix cancer, colon cancer, corpus cancer, endocrine cancer, esophageal cancer, fallopian tube cancer, fat cell cancer, gall bladder cancer, germ cell tumors, gastrointestinal tract cancer, kidney cancer, leukemia, liver cancer, lymphoma, lung cancer, muscle cancer, nervous system cancer, ocular tissue cancer, oral cancer, ovarian cancer, pancreatic cancer, prostate cancer, rectal cancer, skin cancer, small intestine cancer, soft tissue cancer, stomach cancer, teratocarcinoma, testicular cancer, thyroid cancer, ureteral cancer, urinary cancer, uterine cancer, and metastatic cancer of unknown primary site.

15. Use according to Claim 1, wherein the symptoms are selected from the group consisting of abnormal bodily function, abnormal cell morphology, abnormal enzyme levels, abnormal hormone levels, abnormal oncofetal antigen levels, abnormal tissue growth, abnormal tissue mass, abnormal tumor-associated protein levels, altered neurologic function, altered neurologic structure, angiogenesis, bleeding, cells having a cancer cell phenotype, diarrhea, effusions, fatigue, fever, lesions, malnutrition, metastasis, nausea, obstruction of a bodily passageway, opportunistic infection, pain, poor Karnofsky performance status, presence of cell-surface markers, presence of histological markers, presence of intracellular markers, presence of molecular markers, tumor invasion, unregulated cell proliferation, urinary frequency, and weight loss.

16. Use according to Claim 1, wherein the morphogen inhibits cellular incorporation of [³H]-thymidine in an *in vitro* assay.

17. Use according to Claim 1, wherein the medicament further comprises at least one substance in combination with the morphogen.

18. Use according to Claim 17, wherein the substance is an anti-cancer agent.

19. Use according to Claim 1, wherein the cancer cells express at least one bone morphogenic protein receptor (BMPR) specific for the morphogen.

20. Use according to Claim 19, wherein the BMPR is selected from the group consisting of BMPR-IA, BMPR-IB, BMPR-II, and splice variants thereof.

21. Use according to Claim 1, wherein the morphogen reduces tumor size or development.

22. Use according to Claim 1, wherein the morphogen increases survival rate.

23. Use according to Claim 1, wherein the morphogen results in tumor necrosis and diffuse inflammation.

24. Use according to Claim 1, wherein the morphogen is specific for a bone morphogenic protein receptor (BMPR) and the medicament is for treating cancer in a mammal, wherein the mammal expresses at least one of said BMPR.

25. Use according to Claim 24, wherein the cancer is prostate cancer.

26. Use according to Claim 25, wherein the BMPR is selected from the group consisting of BMPR-IA, BMPR-IB, BMPR-II, and splice variants thereof.


SEQUENCE LISTING

<110> Creative Biomolecules, Inc.
      Sampath, et al.

<120> Methods of Alleviating Cancer Symptoms

<130> CRP-166 PCT (00960-521; CBM-21)

<140> filed herewith
<141> 1999-11-12

<160> 9

<170> PatentIn Ver. 2.0

<210> 1
<211> 1822
<212> DNA
<213> Artificial Sequence

<220>
<221> CDS
<222> (49)..(1341)
<223> Osteogenic protein; OP1

<400> 1
ggtgcgggcc cggagcccgg agcccgggta gcgcgtagag ccggcgcg atg cac gtg 57
                                                     Met His Val
                                                       1

cgc tca ctg cga gct gcg gcg ccg cac agc ttc gtg gcg ctc tgg gca 105
Arg Ser Leu Arg Ala Ala Ala Pro His Ser Phe Val Ala Leu Trp Ala
      5                  10                  15

ccc ctg ttc ctg ctg cgc tcc gcc ctg gcc gac ttc agc ctg gac aac 153
Pro Leu Phe Leu Leu Arg Ser Ala Leu Ala Asp Phe Ser Leu Asp Asn
 20                  25                  30                  35

gag gtg cac tcg agc ttc atc cac cgg cgc ctc cgc agc cag gag cgg 201
Glu Val His Ser Ser Phe Ile His Arg Arg Leu Arg Ser Gln Glu Arg
                 40                  45                  50

cgg gag atg cag cgc gag atc ctc tcc att ttg ggc ttg ccc cac cgc 249
Arg Glu Met Gln Arg Glu Ile Leu Ser Ile Leu Gly Leu Pro His Arg
             55                  60                  65

ccg cgc ccg cac ctc cag ggc aag cac aac tcg gca ccc atg ttc atg 297

Pro Arg Pro His Leu Gln Gly Lys His Asn Ser Ala Pro Met Phe Met
        70                  75                  80

ctg gac ctg tac aac gcc atg gcg gtg gag gag ggc ggc ggg ccc ggc 345
Leu Asp Leu Tyr Asn Ala Met Ala Val Glu Glu Gly Gly Gly Pro Gly
    85                  90                  95

ggc cag ggc ttc tcc tac ccc tac aag gcc gtc ttc agt acc cag ggc 393
Gly Gln Gly Phe Ser Tyr Pro Tyr Lys Ala Val Phe Ser Thr Gln Gly
100                 105                 110                 115

ccc cct ctg gcc agc ctg caa gat agc cat ttc ctc acc gac gcc gac 441
Pro Pro Leu Ala Ser Leu Gln Asp Ser His Phe Leu Thr Asp Ala Asp
                120                 125                 130

atg gtc atg agc ttc gtc aac ctc gtg gaa cat gac aag gaa ttc ttc 489
Met Val Met Ser Phe Val Asn Leu Val Glu His Asp Lys Glu Phe Phe
            135                 140                 145

cac cca cgc tac cac cat cga gag ttc cgg ttt gat ctt tcc aag atc 537
His Pro Arg Tyr His His Arg Glu Phe Arg Phe Asp Leu Ser Lys Ile
        150                 155                 160

cca gaa ggg gaa gct gtc acg gca gcc gaa ttc cgg atc tac aag gac 585
Pro Glu Gly Glu Ala Val Thr Ala Ala Glu Phe Arg Ile Tyr Lys Asp
    165                 170                 175

tac atc cgg gaa cgc ttc gac aat gag acg ttc cgg atc agc gtt tat 633
Tyr Ile Arg Glu Arg Phe Asp Asn Glu Thr Phe Arg Ile Ser Val Tyr
180                 185                 190                 195

cag gtg ctc cag gag cac ttg ggc agg gaa tcg gat ctc ttc ctg ctc 681
Gln Val Leu Gln Glu His Leu Gly Arg Glu Ser Asp Leu Phe Leu Leu
                200                 205                 210

gac agc cgt acc ctc tgg gcc tcg gag gag ggc tgg ctg gtg ttt gac 729
Asp Ser Arg Thr Leu Trp Ala Ser Glu Glu Gly Trp Leu Val Phe Asp
            215                 220                 225

atc aca gcc acc agc aac cac tgg gtg gtc aat ccg cgg cac aac ctg 777
Ile Thr Ala Thr Ser Asn His Trp Val Val Asn Pro Arg His Asn Leu
        230                 235                 240

ggc ctg cag ctc tcg gtg gag acg ctg gat ggg cag agc atc aac ccc 825
Gly Leu Gln Leu Ser Val Glu Thr Leu Asp Gly Gln Ser Ile Asn Pro
    245                 250                 255

aag ttg gcg ggc ctg att ggg cgg cac ggg ccc cag aac aag cag ccc 873

Lys Leu Ala Gly Leu Ile Gly Arg His Gly Pro Gln Asn Lys Gln Pro
260                 265                 270                 275

ttc atg gtg gct ttc ttc aag gcc acg gag gtc cac ttc cgc agc atc     921
Phe Met Val Ala Phe Phe Lys Ala Thr Glu Val His Phe Arg Ser Ile
                280                 285                 290

cgg tcc acg ggg agc aaa cag cgc agc cag aac cgc tcc aag acg ccc     969
Arg Ser Thr Gly Ser Lys Gln Arg Ser Gln Asn Arg Ser Lys Thr Pro
            295                 300                 305

aag aac cag gaa gcc ctg cgg atg gcc aac gtg gca gag aac agc agc     1017
Lys Asn Gln Glu Ala Leu Arg Met Ala Asn Val Ala Glu Asn Ser Ser
        310                 315                 320

agc gac cag agg cag gcc tgt aag aag cac gag ctg tat gtc agc ttc     1065
Ser Asp Gln Arg Gln Ala Cys Lys Lys His Glu Leu Tyr Val Ser Phe
    325                 330                 335

cga gac ctg ggc tgg cag gac tgg atc atc gcg cct gaa ggc tac gcc     1113
Arg Asp Leu Gly Trp Gln Asp Trp Ile Ile Ala Pro Glu Gly Tyr Ala
340                 345                 350                 355

gcc tac tac tgt gag ggg gag tgt gcc ttc cct ctg aac tcc tac atg     1161
Ala Tyr Tyr Cys Glu Gly Glu Cys Ala Phe Pro Leu Asn Ser Tyr Met
                360                 365                 370

aac gcc acc aac cac gcc atc gtg cag acg ctg gtc cac ttc atc aac     1209
Asn Ala Thr Asn His Ala Ile Val Gln Thr Leu Val His Phe Ile Asn
            375                 380                 385

ccg gaa acg gtg ccc aag ccc tgc tgt gcg ccc acg cag ctc aat gcc     1257
Pro Glu Thr Val Pro Lys Pro Cys Cys Ala Pro Thr Gln Leu Asn Ala
        390                 395                 400

atc tcc gtc ctc tac ttc gat gac agc tcc aac gtc atc ctg aag aaa     1305
Ile Ser Val Leu Tyr Phe Asp Asp Ser Ser Asn Val Ile Leu Lys Lys
    405                 410                 415

tac aga aac atg gtg gtc cgg gcc tgt ggc tgc cac tagctcctcc          1351
Tyr Arg Asn Met Val Val Arg Ala Cys Gly Cys His
420                 425                 430

gagaattcag accctttggg gccaagtttt tctggatcct ccattgctcg ccttggccag 1411

gaaccagcag accaactgcc ttttgtgaga ccttcccctc cctatcccca actttaaagg 1471

tgtgagagta ttaggaaaca tgagcagcat atggcttttg atcagttttt cagtggcagc 1531

atccaatgaa caagatccta caagctgtgc aggcaaaacc tagcaggaaa aaaaaacaac 1591

gcataaagaa aaatggccgg gccaggtcat tggctgggaa gtctcagcca tgcacggact 1651

cgtttccaga ggtaattatg agcgcctacc agccaggcca cccagccgtg ggaggaaggg 1711

ggcgtggcaa ggggtgggca cattggtgtc tgtgcgaaag gaaaattgac ccggaagttc 1771

ctgtaataaa tgtcacaata aaacgaatga atgaaaaaaa aaaaaaaaaa a 1822

<210> 2
<211> 431
<212> PRT
<213> Artificial Sequence

<400> 2
Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

<210> 3
<211> 102
<212> PRT
<213> Artificial Sequence

<220>
<221> PEPTIDE
<222> (1)..(102)
<223> OPX; where each Xaa is independently selected from
      a group of one or more specified amino acids as
      defined in the specification

<400> 3
Cys Xaa Xaa His Glu Leu Tyr Val Ser Phe Xaa Asp Leu Gly Trp Xaa
1               5                   10                  15

Asp Trp Xaa Ile Ala Pro Xaa Gly Tyr Xaa Ala Tyr Tyr Cys Glu Gly
            20                  25                  30

Glu Cys Xaa Phe Pro Leu Xaa Ser Xaa Met Asn Ala Thr Asn His Ala
        35                  40                  45

Ile Xaa Gln Xaa Leu Val His Xaa Xaa Xaa Pro Xaa Xaa Val Pro Lys
    50                  55                  60

Xaa Cys Cys Ala Pro Thr Xaa Leu Xaa Ala Xaa Ser Val Leu Tyr Xaa
65                  70                  75                  80

Asp Xaa Ser Xaa Asn Val Ile Leu Xaa Lys Xaa Arg Asn Met Val Val
                85                  90                  95

Xaa Ala Cys Gly Cys His
            100

<210> 4
<211> 97
<212> PRT
<213> Artificial Sequence

<220>
<221> PEPTIDE
<222> (1)..(97)
<223> Generic-Seq-7; wherein each Xaa is independently

selected from a group of one or more specified
amino acids as defined in the specification

<400> 4
Leu Xaa Xaa Xaa Phe Xaa Xaa Xaa Gly Trp Xaa Xaa Xaa Xaa Xaa Xaa
1               5                   10                  15

Pro Xaa Xaa Xaa Xaa Ala Xaa Tyr Cys Xaa Gly Xaa Cys Xaa Xaa Pro
            20                  25                  30

Xaa Xaa Xaa Xaa Xaa Xaa Xaa Xaa Asn His Ala Xaa Xaa Xaa Xaa Xaa
        35                  40                  45

Xaa Xaa Xaa Xaa Xaa Xaa Xaa Xaa Xaa Xaa Xaa Xaa Cys Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Val Xaa Leu Xaa Xaa Xaa Xaa Xaa Met Xaa Val Xaa Xaa Cys Xaa Cys
                85                  90                  95

Xaa

<210> 5
<211> 102
<212> PRT
<213> Artificial Sequence

<220>
<221> PEPTIDE
<222> (1)..(102)
<223> Generic-Seq-8; wherein each Xaa is independently
      selected from a group of one or more specified
      amino acids as defined in the specification

<400> 5
Cys Xaa Xaa Xaa Xaa Leu Xaa Xaa Xaa Phe Xaa Xaa Xaa Gly Trp Xaa
1               5                   10                  15

Xaa Xaa Xaa Xaa Xaa Pro Xaa Xaa Xaa Xaa Ala Xaa Tyr Cys Xaa Gly
            20                  25                  30

Xaa Cys Xaa Xaa Pro Xaa Xaa Xaa Xaa Xaa Xaa Xaa Xaa Asn His Ala
        35                  40                  45

Xaa Xaa Xaa Xaa Xaa Xaa Xaa Xaa Xaa Xaa Xaa Xaa Xaa Xaa Xaa Xaa
    50                  55                  60

Xaa Cys Cys Xaa Pro Xaa Xaa Xaa Xaa Xaa Xaa Xaa Xaa Leu Xaa Xaa
65                  70                  75                  80

Xaa Xaa Xaa Xaa Xaa Val Xaa Leu Xaa Xaa Xaa Xaa Xaa Met Xaa Val
                85                  90                  95

Xaa Xaa Cys Xaa Cys Xaa
            100

<210> 6
<211> 97
<212> PRT
<213> Artificial Sequence

<220>
<221> PEPTIDE
<222> (1)..(97)
<223> Generic-Seq-9; wherein each Xaa is independently
selected from a group of one or more specified
amino acids as defined in the specification

<400> 6
Xaa Xaa Xaa Xaa Xaa Xaa Xaa Xaa Xaa Xaa Xaa Xaa Xaa Xaa Xaa Xaa
1               5                   10                  15

Pro Xaa Xaa Xaa Xaa Xaa Xaa Xaa Cys Xaa Gly Xaa Cys Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Cys Xaa Cys
                85                  90                  95

Xaa

<210> 7
<211> 102
<212> PRT
<213> Artificial Sequence

<220>
<221> PEPTIDE
<222> (1)..(102)
<223> Generic-Seq-10;  wherein each Xaa is independently
      selected from a group of one or more specified
      amino acids as defined in the specification

<400> 7
Cys Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Pro Xaa Xaa Xaa Xaa Xaa Xaa Xaa Cys Xaa Gly
            20                  25                  30

Xaa Cys Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Cys Xaa Pro Xaa Xaa Xaa Xaa Xaa Xaa Xaa Xaa Leu Xaa Xaa
65                  70                  75                  80

Xaa Xaa Xaa Xaa Xaa Xaa Xaa Xaa Xaa Xaa Xaa Xaa Xaa Xaa Xaa Xaa
                85                  90                  95

Xaa Xaa Cys Xaa Cys Xaa
            100


<210> 8
<211> 5
<212> PRT
<213> Artificial Sequence

<220>
<221> PEPTIDE
<222> (1)..(5)
<223> wherein each Xaa is independently selected from a
      group of one or more specified amino acids as
      defined in the specification

<400> 8

Cys Xaa Xaa Xaa Xaa
1               5

<210> 9
<211> 5
<212> PRT
<213> Artificial Sequence

<220>
<221> PEPTIDE
<222> (1)..(5)
<223> wherein each Xaa is independently selected from a
      group of one or more specified amino acids as
      defined in the specification

<400> 9
Cys Xaa Xaa Xaa Xaa
1               5